# EUROPEAN PATENT APPLICATION

(11) **EP 4 600 357 A1**
(43) Date of publication of application: **13.08.2025**
(21) Application number: 24157212.2
(22) Date of filing: 12.02.2024
(51) Int. Cl.: C12N 15/113, C07K 14/005

(54) **DELIVERY SYSTEM FOR GENE EDITING TOOLS**

(71) Applicant: Technische Universität München, 80333 München (DE); Helmholtz Zentrum München Deutsches Forschungszentrum für Gesundheit und Umwelt GmbH, 85764 Neuherberg (DE)
(72) Inventor: The designation of the inventor has not yet been filed
(74) Representative: Engelhard, Markus

(57) **Abstract**

The present invention relates to a protein-ribonucleic acid complex comprising (I) at least one multi-functional protein or a set of at least two proteins and (II) a nucleic acid editor complex, a polynucleotide encoding the multi-functional protein (I) and its uses in the delivery of nucleic acids and gene editing tools. The present invention also relates to a nucleic acid delivery system, a plurality of polynucleotides, pharmaceutical compositions, a cell or a cell line, and kits. The present invention further relates to medical uses of the nucleic acid delivery system or the cell or cell line. Also provided are uses of a cell or a cell line in therapeutic and diagnostic applications, as well as in tissue engineering. Finally, the present invention further relates to an *in vitro* or *in vivo* method of editing a nucleic acid molecule in a target cell, and a method for nucleic acid delivery.

## Description

The present invention relates to a protein-ribonucleic acid complex comprising (I) at least one multi-functional protein or a set of at least two proteins and (II) a nucleic acid editor complex, a polynucleotide encoding the multi-functional protein (I) and its uses in the delivery of nucleic acids and gene editing tools. The present invention also relates to a nucleic acid delivery system, a plurality of polynucleotides, pharmaceutical compositions, a cell or a cell line, and kits. The present invention further relates to medical uses of the nucleic acid delivery system or the cell or cell line. Also provided are uses of a cell or a cell line in therapeutic and diagnostic applications, as well as in tissue engineering. Finally, the present invention further relates to an *in vitro* or *in vivo* method of editing a nucleic acid molecule in a target cell, and a method for nucleic acid delivery.

### BACKGROUND OF THE INVENTION

The fast-paced developments of various gene editing technologies in the last decade have revolutionized the field of molecular biology, enabling the precise manipulation of virtually any gene with single-nucleotide precision. Especially the recent development of base editors (BEs) (Komor *et al.,* 2016) and prime editors (PEs) (Anzalone *et al.,* 2019), mediating single-nucleotide conversions, deletion/insertions (Anzalone *et al.,* 2022; Choi *et al.,* 2022), or even whole gene insertions (Yarnall *et al.,* 2022) all without the necessity of highly mutagenic double-stranded DNA breaks (DSBs), represent a milestone towards the ultimate goal of curing genetic disorders. However, efficient and safe delivery of the respective editing complexes remains a challenge.

Viral vector-based systems have emerged as a promising method for delivering genome editing machineries. However, despite the advances in this field, there remains a need for improved efficiency and specificity to maximize the potential of these delivery systems. While adeno-associated viruses (AAVs) are considered safe and efficient for *in vivo* gene delivery, they also face limitations that hinder their broad application in gene editing. These limitations include constrained cargo capacity, potential immunogenicity, and the risk of insertional mutagenesis. Importantly, while their long persistence is usually regarded as a benefit, for the same reason, AAV-based delivery is presumably not ideally suited for certain prime editing strategies. Third-generation prime editing system PE3(b) uses a nicking guide RNA (ngRNA) in addition to the primary prime editing guide RNA (pegRNA) to stimulate the intended edit being incorporated. The long persistence of AAVs, when used for PE3(b) strategies, could lead to long-term continuous nicking events even after the desired edit has been incorporated. Consequently, the risk of unintended mutations at the nick site is considerably higher, and the permanent nick might lead the DNA repair machinery to occupy that site, potentially repressing the transcription of the gene intended for repair.

Lentiviral vectors have been employed in the generation of chimeric antigen receptor (CAR) T cells, modified to target and eliminate cancer cells, ultimately resulting in FDA-approved CAR-T cell therapies. However, using lentiviral vectors to deliver gene editing complexes raises several severe safety concerns. Lentiviruses integrate their genetic payload into the host genome, providing stable and long-term transgene expression, which is problematic when delivering gene editing machineries, as the persistent expression may increase the risk of undesired on- and off-target effects. The random integration of lentiviral vectors into the host genome also raises safety concerns since their integration can disrupt essential endogenous genes or regulatory elements, potentially leading to oncogenesis.

In addition, both AAV-based and lentiviral vector systems pose the risk of high immunogenicity due to their persistent expression of the editing machinery. Consequently, repaired cells are highly likely to be rejected by the host's immune system.

Lipid nanoparticles (LNPs) on the other hand have gained attention as non-viral delivery vehicles for nucleic acids and are often favored over AAVs due to their packaging capacity and range of therapeutic applications. However, they often exhibit limited cell-type specificity, potential cytotoxicity, and may require complex formulation optimization for efficient delivery.

In contrast, virus-like particle (VLP) systems offer several advantages, including the ability to package and deliver genome editing complexes without the risk of insertional mutagenesis or the activation of host immune responses due to viral components or persistent transgene expression. VLPs can be easily pseudotyped, enabling the modulation of cell or tissue tropism by utilizing the diversity of known viruses with different tropisms. This feature allows for the reengineering of glycoproteins to target specific cell types or tissues, further enhancing the versatility and applicability of VLP systems.

### SUMMARY OF THE INVENTION

According to the present invention this object is solved by a polynucleotide encoding the multi-functional fusion protein of the present invention.

According to the present invention this object is solved by a complex of at least one multi-functional fusion protein of the present invention with a specific mRNA, sgRNA or pegRNA; or with a gene-editing ribonucleoprotein (RNP) complex comprising a specific mRNA, sgRNA or pegRNA.

According to the present invention this object is solved by using the multi-functional fusion protein of the present invention and/or the polynucleotide encoding it for:
- generating a nucleic acid delivery system, preferably a virus-like particle (VLP),
- delivering nucleic acids, preferably gene-editing nucleic acids, epigenome modulator nucleic acids, transcriptome-editing nucleic acids, and/or epitranscriptome modulator nucleic acids.

According to the present invention this object is solved by a nucleic acid delivery system, said nucleic acid delivery system comprising:
*(A)* at least one multi-functional fusion protein of the present invention;
*(B)* a gene-editing ribonucleoprotein (RNP) complex, which comprises
*(B1)* an mRNA, a non-coding RNA (ncRNA), or a guide RNA, such as sgRNA or pegRNA, which is tagged by a nucleic acid binding domain; and
*(B2)* gene-editing polypeptide(s) or protein(s),
*(C)* a viral envelope protein or engineered variants thereof,
*(D)* a targeting domain, such as a glycoprotein or engineered variants thereof,
*(E)* a moiety mediating endosomal escape, such as a glycoprotein or engineered variants thereof,
*(F)* optionally, a reporter gene product such as a luciferase or a fluorescent protein, and
(*G*) optionally, a nucleic acid-stabilizing protein, preferably Csy4.

In one embodiment, the gene-editing ribonucleoprotein (RNP) complex (*B*) is not a protein fusion to gag. Instead, an indirect transport mechanism occurs via the guide RNA listed in (*B1*)*.*

According to the present invention this object is solved by a plurality of polynucleotides, comprising
*(1)* a first polynucleotide comprising a nucleic acid sequence encoding a multi-functional fusion protein as defined herein;
*(2)* a second polynucleotide comprising a nucleic acid sequence encoding a gene editor protein;
*(3)* a third polynucleotide comprising a nucleic acid sequence encoding a guide RNA, such as a sgRNA or pegRNA, which is optionally tagged by a nucleic acid binding domain; and
*(4)* a fourth polynucleotide comprising a nucleic acid sequence encoding an envelope protein.

According to the present invention this object is solved by one or more vectors comprising the plurality of polynucleotides of the present invention, wherein the one or more vectors are preferably three vectors.

According to the present invention this object is solved by a pharmaceutical composition comprising:
the polynucleotide of the present invention which encodes the fusion protein of the present invention, the nucleic acid delivery system of the present invention, the plurality of polynucleotides of the present invention or the one or more vectors of the present invention;
optionally, pharmaceutically acceptable excipients and/or carrier.

According to the present invention this object is solved by a cell or cell line comprising the fusion protein of the present invention, the polynucleotide of the present invention, the nucleic acid delivery system of the present invention, the plurality of polynucleotides of the present invention or the one or more vectors of the present invention.

According to the present invention this object is solved by a kit comprising:
the polynucleotide of the present invention which encodes the fusion protein of the present invention, or
the nucleic acid delivery system of the present invention, or
the plurality of polynucleotides of the present invention, or
the one or more vectors of the present invention, or
the cell or cell line of the present invention.

According to the present invention this object is solved by a nucleic acid delivery system, preferably a virus-like particle, produced by means of transfection, lipofection, electroporation, photoporation, sonoporation, particle bombardment, microinjection, magnetofection, transduction, such as viral transduction, cell-penetrating peptides, calcium-phosphate, nanoparticles, combinations thereof, or otherwise inserting the plurality of polynucleotides of the present invention or the one or more vectors of the present invention into a cell and expressing the components of the nucleic acid delivery system from the plurality of polynucleotides or the one or more vectors of the present invention in the cell, thereby allowing the nucleic acid delivery system, preferably the virus-like particle, to assemble, such as spontaneously assemble, in the cell.

According to the present invention this object is solved by providing the nucleic acid delivery system of the present invention for use in medicine.

According to the present invention this object is solved by providing the nucleic acid delivery system of the present invention for use in the treatment of genetic diseases, preferably monogenetic diseases and/or polygenetic diseases.

According to the present invention this object is solved by an *in vitro* or *in vivo* method of editing a nucleic acid molecule in a target cell by nuclease mediated editing, base editing or prime editing, or homology directed repair, such as by co-delivery of an integrase-deficient (D64V) lentivirus (IDLV), wherein, preferably, the IDLV is packaged using a psi-NC interaction, a PP7-PCP, MS2-MCP, and/or a C4-Csy4 interaction, comprising:
contacting the target cell with a nucleic acid delivery system of the present invention, or with the pharmaceutical composition of the present invention,
thereby installing one or more modifications to the nucleic acid at a target site.

According to the present invention this object is solved by a method for nucleic acid delivery, preferably gene-editing nucleic acids. Said method comprises the steps of:
(I) providing a multi-functional fusion protein of the present invention to a cell, wherein said cell produces in its nucleus a gene-editing ribonucleoprotein (RNP) complex, which comprises an mRNA or a guide RNA, such as a sgRNA or pegRNA, wherein said gene-editing ribonucleoprotein (RNP) complex is as defined herein;
(II) allowing the multi-functional fusion protein to locate to the nucleus of said cell;
(III) forming a complex of the multi-functional fusion protein with the gene-editing ribonucleoprotein (RNP) complex in the nucleus;
(IV) allowing the complex of (III) to be exported to the cytosol of the cell;
(V) budding of virus-like particles comprising the gene-editing ribonucleoprotein (RNP) complex;
(VI) binding to a target cell surface, preferentially via a specific receptor;
(VII) internalization into a target cell, preferentially via endocytosis;
(VIII) transport into the cytosol, e.g. via endosomal escape;
(IX) dissociation of the RNP (and optionally bound `nucleic acid editing complex) from the nucleic acid binding domain;
(X) optionally transport into the nucleus for genome editing or transactivation, and
(X) optionally binding of the guide-RNA-editing complex to an RNA target sequence.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 20" should be interpreted to include not only the explicitly recited values of 1 to 20, but also include individual values and sub-ranges within the indicated range. Thus, included in this numerical range are individual values such as 1, 2, 3, 4, 5 .... 17, 18, 19, 20 and sub-ranges such as from 2 to 10, 8 to 15, etc. This same principle applies to ranges reciting only one numerical value, such as "higher than 100". Furthermore, such an interpretation should apply regardless of the breadth of the range or the characteristics being described.

### Multi-functional fusion protein, its encoding nucleic acid and its uses

As discussed above, the present invention provides a multi-functional fusion protein, such as a protein-ribonucleic acid complex comprising:
(I) at least one multi-functional protein or a set of at least two proteins, wherein the at least one multi-functional protein or the set of at least two proteins comprise the following protein domains:
   *(a)* at least one plasma membrane-interacting domain;
   *(b)* at least one oligomerization domain;
   *(c)* a membrane-bending domain;
   *(d)* at least one shuttling domain comprised of at least one nuclear export signal (NES) and
      nuclear localization signal (NLS);
   *(e)* a nucleic acid-binding (NAB) domain, capable of binding a single strand or double-strand RNA or DNA motif; and
   *(f)* a budding domain, which comprises a monopartite or multipartite motif that enables ESCRT-dependent or ESCRT-independent budding;
   *(g)* optionally, a reverse transcriptase domain;
   *(h)* optionally, a maturation factor;
   *(i)* optionally, a reporter-signal generating domain;
   *(j)* optionally one nucleic acid-stabilizing domain and
(II) a nucleic acid editor complex, which comprises an mRNA, a guide RNA or a ncRNA,
which is bound via the nucleic acid-binding domain *(e)* of (I), preferably as ribonucleoprotein complex, wherein only the RNA species comprises the nucleic acid motif, bound by NAB domain *(e)* of (I), wherein the RNA is preferentially a gRNA, and wherein the RNA species comprises optionally an autonomous 3'-stabilization motif and/or a motif that is protected by an additional heterologously expressed nucleic acid-stabilizing domain *(*j) of (I).

In one embodiment, the nucleic acid-binding (NAB) domain *(e)* of (I) is capable of binding a specific single stranded or double-stranded RNA or DNA motif.

The multi-functional protein of the present invention is designed to:
(1) shuttle between the nucleus and cytosol,
(2) bind to specific nucleic acids in the cell,
(3) bud off the plasma membrane of a packaging/sender cell along with specific nucleic acids and co-packaged proteinaceous components, and
(4) internalize into a recipient/target cell, delivering the specific nucleic acids, or co-delivering specific nucleic acid and proteinaceous components for cellular processing, such as gene editing.

### - Plasma membrane-interacting domain (a)

The multi-functional fusion protein of the present invention comprises at least one plasma membrane-interacting domain *(a)*.

Said plasma membrane-interacting domain *(a)* is preferably a lipid group. A preferred lipid group is/are C12 to C18 fatty acid(s), preferably myristoyl (C14), palmitoyl (C16), and/or isoprenoid(s), preferably C-farnesyl. In one embodiment, the myristoyl moiety is N-terminal or near the N-terminus. In one embodiment, the farnesyl moiety is C-terminal or near the C-terminus. In one embodiment, the palmitoylation is an S-palmitoylation. In one embodiment, palmitoylation is an O- or N-palmitoylation.

In one embodiment, the plasma membrane-interacting domain *(a)* is preferably a polypeptide domain capable of binding membrane proteins or membrane components, which are, for example, fatty acid chains, phospholipids, glycolipids, phosphorglycerids, sphingolipids, sterols. In one embodiment, said polypeptide domain is a pleckstrin homology (PH) domain, such as phospholipase C-δ1 pleckstrin homology domains (PH).

Further such polypeptide domains are C2 domain, FYVE domain, phox homology (PX) domains, PHD finger domain, PROPPINs (β-propellers that bind polyphosphoinositides) domain, epsin N-terminal homology (ENTH) domain, BAR (Bin-Amphiphysin-Rvs) domains, Tubby domain.

The skilled artisan knows further plasma membrane-interacting domains.

### - Oligomerization domain (b)

The multi-functional fusion protein of the present invention comprises at least one oligomerization domain *(b).*

The at least one oligomerization domain *(b)* preferably enables homo- or hetero-multimerization of one or multiple polypeptide species.

The multi-functional protein of the present invention preferably forms dimers.

The at least one oligomerization domain *(b)* enables homo-and/or hetero multimerizing, such as dimer, trimer, tetramer, etc.

Preferably, the oligomerization domain (b) comprises or contains coiled coils or PDZ domains. In one embodiment, the oligomerization domain (b) comprises or contains *de novo* generated homo/hetero multimerization domains composed of coil-only, sheet-only, or mixed-type proteins.

### - Membrane-bending domain (c)

The multi-functional fusion protein of the present invention comprises a membrane-bending or a curvature-forming domain *(c).*

The membrane-bending domain *(c)* preferably induces plasma membrane bending and formation of nanospheres with a diameter, preferably in the range from about 50 nm to about 1 µm.

In one embodiment, the membrane-bending domain *(c)* is a structural polyprotein, such as the HIV-1 gag polyprotein, and/or modified/engineered/truncated variants thereof.

### - Nuclear-cytosolic shuttling signal (d)

The multi-functional fusion protein of the present invention comprises at least one
nuclear export signal (NES), and
nuclear localization signal (NLS);

The NES facilitates the export of the multi-functional protein towards the cytosol when the multi-functional protein is localized in the nucleus.

In one embodiment, the NES *(d)* is the NES of HIV-1 (NES_{HIV-1}), preferably wherein the NES comprises an amino acid sequence according to SEQ ID NO: 65. In one embodiment, the NES *(d)* is the NES of DBR1.

The NLS is a mono- or bipartite NLS and facilitates the import of the multi-functional fusion protein towards the nucleus when the multi-functional protein is localized in the cytosol.

In one embodiment, the NLS *(d)* of (I) is the NLS of c-Myc protein (NLS_{c-Myc}), NLS of SV40 (NLS_{SV40}) or a synthetic NLS, such as attenuated variants of NLS_{c-Myc} and NLS_{SV40}, or other mono- or bipartite synthetic NLS, preferably wherein the NLS comprises an amino acid sequence according to any one of SEQ ID NO: 48 to 54.

The inventors surprisingly found that the combination of an NES together with an NLS in one protein, namely the multi-functional fusion protein (I) of the present invention, enables the protein's specific shuttling functionality where it shuttles between the nucleus and the cytosol and enables thereby the binding of its RNA/RNP cargo in the nucleus and the transport towards the cell membrane. The inventors are the first ones to show that by introducing the shuttling NLS/NES gag in the context of an RNP-editor loading to VLPs, the editing efficacy of the resulting RNP-VLPs can strongly be increased.

In a preferred embodiment, the NES and NLS together form a shuttling motif.

For example, NLS-NES-NLS_{FLAG}, wherein NLS_{FLAG} refers to a synthetic NLS,
preferably NLS_{c-Myc}-NES_{HIV-1}-NLS_{FLAG}.

### - Nucleic acid-binding domain (e)

The multi-functional fusion protein of the present invention comprises at least one nucleic acid-binding domain *(e).*

Said nucleic acid-binding domain *(e)* is capable of binding single strand or double-strand RNA or DNA, namely through specific interactions, including recognition of linear motifs, specific secondary structures, tertiary structures, or combinations thereof.

Preferably, nucleic acid-binding domain *(e)* is capable of binding specific RNA molecules, including mRNA, guide RNA (such as sgRNA, pegRNA), and DNA, which can be tagged with specific RNA aptamer motifs.

In one embodiment, the nucleic acid-binding domain *(e)* of (I) is a C4-aptamer binding protein (Csy4-enzyme) domain, preferably a Csy4 domain comprising an amino acid sequence according to SEQ ID NO: 64. In one embodiment, the nucleic acid-binding domain *(e)* of (I) is an RNA-binding PP7 coat protein (PCP) domain, optionally single chain tandem PCP, preferably a PCP domain comprising an amino acid sequence according to SEQ ID NO: 77, 78, or 79. In one embodiment, the nucleic acid-binding domain *(e)* of (I) is an MS2 coat protein (MCP) domain, preferably a MCP domain comprising an amino acid sequence according to SEQ ID NO: 58, or a single chain tandem MCP. In one embodiment, the nucleic acid-binding domain *(e)* of (I) is a boxB domain or the N Protein of bacteriophage P22.

In one embodiment, the nucleic acid-binding domain *(e)* of (I) is a Com-com, which is a Com protein that binds to the structure of a com RNA hairpin, as described in Zalatan *et al.* (2014). In one embodiment, the nucleic acid-binding domain *(e)* of (I) a psi-binding nucleocapsid (NC) domain of HIV-1 Gag. The NC domain of HIV-1 gag can be used to package psi-tagged RNAs. All aptamer binding protein domains can be arranged as single chain tandem protein.

### - Budding domain (f)

The multi-functional fusion protein of the present invention comprises a budding domain *(g).* Said budding domain preferably comprises a monopartite or multipartite motif that enables ESCRT-dependent or ESCRT-independent budding.

In one embodiment, the budding domain *(f)* of (I) is a HIV-1 p6 domain, preferably a p6 domain comprising an amino acid sequence according to SEQ ID NO: 75, an equine infectious anemia virus (EIAV) p9 domain, a murine leukaemia virus (MLV) p12 domain, a rouse sarcoma virus (RSV) or human T-cell leukemia virus type 1 (HTLV-1) p19 domain, a retroviral L domain, a vesicular stromatitis virs (VSV) M protein, an influenza virus M2 protein, an alphavirus F 13 protein, or an arenavirus Z protein.

### - optionally, a reverse transcriptase domain (g)

The multi-functional fusion protein of the present invention optionally comprises a reverse transcriptase domain *(g).*

In one embodiment, the RT domain *(g)* of (I) is pol of HIV-1 or a mutant thereof, wherein the enzymatic function of the integrase domain is inactivated by mutating at least one of the amino acids of pol of HIV-1 at position D64 (such as pol D64V), D116, E152, and/or F185 (i.e. the key amino acids of pol of HIV-1).

### - optionally, a maturation factor (h)

The multi-functional fusion protein of the present invention optionally comprises a maturation factor *(h).*

Importantly, the inventors found a way to modularly package the RNP via the guide RNA and maturation of gag occurs much more naturally compared to prior work where the Cas component is directly fused to the gag as a fusion protein and its release relies on an additional protease cleavage site. Packaging via the guide RNA and maturation of gag according to this invention is thus much simpler and more modular since any RNP, and any Cas protein that uses a guide RNA (e.g., Cas9, Cas12, Cas13) can be packaged without the need to optimize the protein fusion, such as the linker and the proteolytic site, compared to prior work where the Cas component is directly fused to gag as fusion protein. Also in the case where coiled-coiled adapters are used instead of a direct fusion to gag, the guide-RNA mediated packaging is superior to the Cas-protein-mediated packaging of the editor RNP complex because the integrity of the RNP component with the least stability and shortest half-life time (i.e. the guide RNA component) is used as the handle, thereby ensuring that fully functional RNP editor complexes are loaded instead of Cas proteins without functional guideRNA.

Importantly, the inventors further discover that maturation of gag is dispensable, as shown using mini-Gag constructs. The inventors further discovered that the dissociation of the RNP-payload occurs naturally due to the non-covalent packaging. Moreover, the dissociation of the RNP-payload is not dependent on proteolysis.

In one embodiment, the maturation factor *(h)* of (I) is an enzyme capable of cleaving the multi-functional fusion protein of the present invention into multiple functional domains, preferably when it is interacting with the membrane.

In one embodiment, the maturation factor *(h)* is the HIV-1 protease.

In one embodiment, at least two maturation sites upstream and downstream of the NAB domain *(e)* are present.

Preferably, the multi-functional fusion protein of the present invention does not contain a proteolytic activity. In one embodiment, the release of the ribonucleoprotein complex does not rely on proteolytic maturation of the fusion protein. In one embodiment, the multi-functional fusion protein of the present invention optionally comprises proteolytic activity to enhance infectivity.

Importantly, the inventors found a mechanism that dissociates Gag maturation from RNP-packaging, thus making the functionality (4), i.e. simultaneous co-packaging and delivery of protein components modular gene editors without the need for further modification (e.g., fusion or proteolytic activation) of the gene editor proteins highly beneficial over prior art work.

### - optionally, a reporter-signal generating domain (i)

The multi-functional fusion protein of the present invention optionally comprises a reporter-signal generating domain *(i).*

In one embodiment, the reporter-signal generating domain *(i)* of (I) is fluorescence, bioluminescence, photoabsorption or photoacoustic detection, detection by Nuclear Magnetic Resonance, Positron emission tomography, as well as read out via biochemical methods, RNA or DNA detection methods such as RT-qPCR, qPCR, or next-generation sequencing.

### - optionally a nucleic acid-stabilizing domain (j)

The multi-functional fusion protein or a set of at least two proteins of the present invention optionally comprise a nucleic acid-stabilizing domain *(j)* to specifically bind to an aptamer motif on a pegRNA whereby the pegRNA is stabilized and protected.

In one embodiment, the set of at least two proteins of the present invention are 2 to 10 proteins, such as 2 to 5 proteins.

In one embodiment, the nucleic acid-stabilizing domain *(j)* is the Csy4/Cas6f protein in combination with the C4 aptamer on the 3' of the pegRNA. The Csy4/C4 combination can be used for RNP packaging, while simultaneously processing and stabilizing the pegRNA to enhance prime editing (PE).

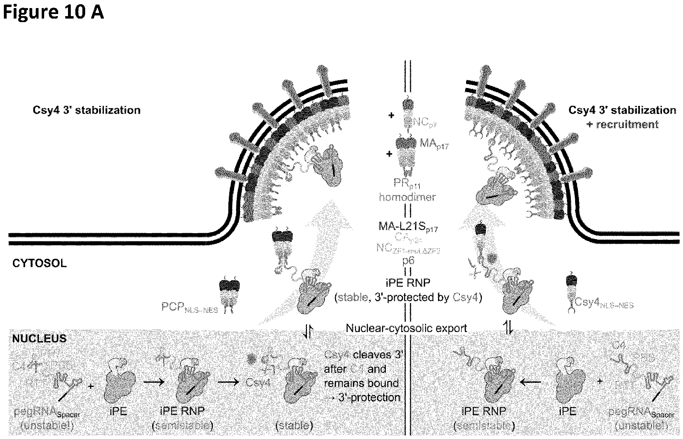

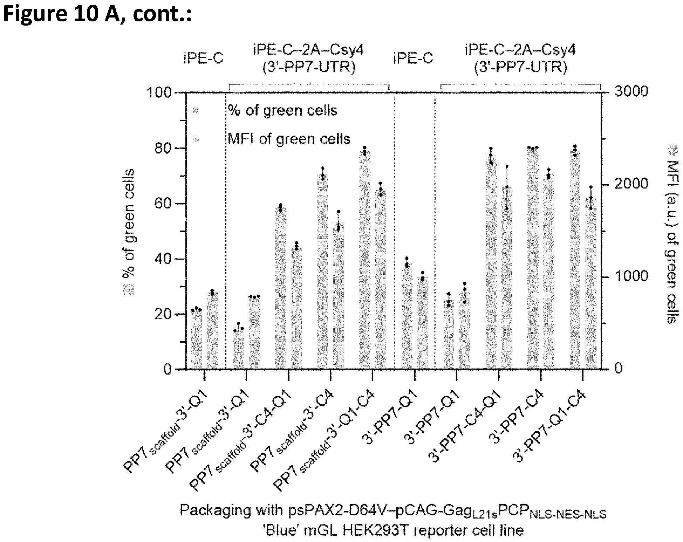

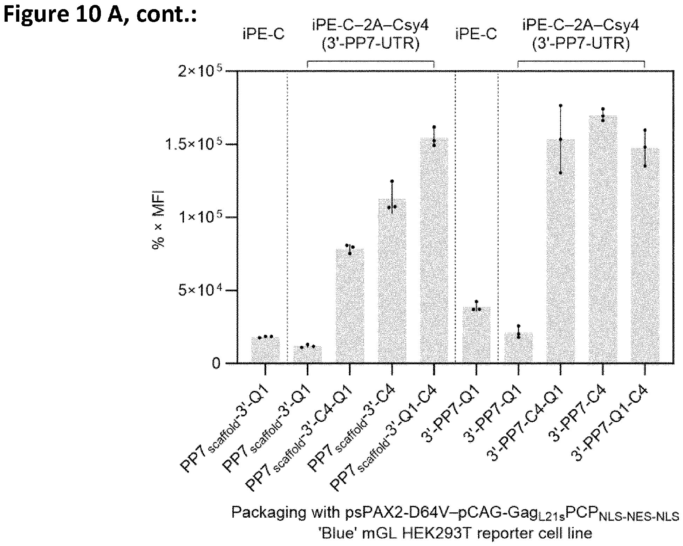

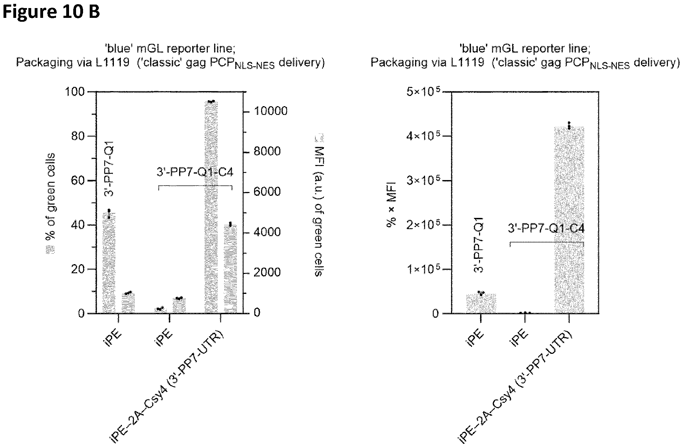

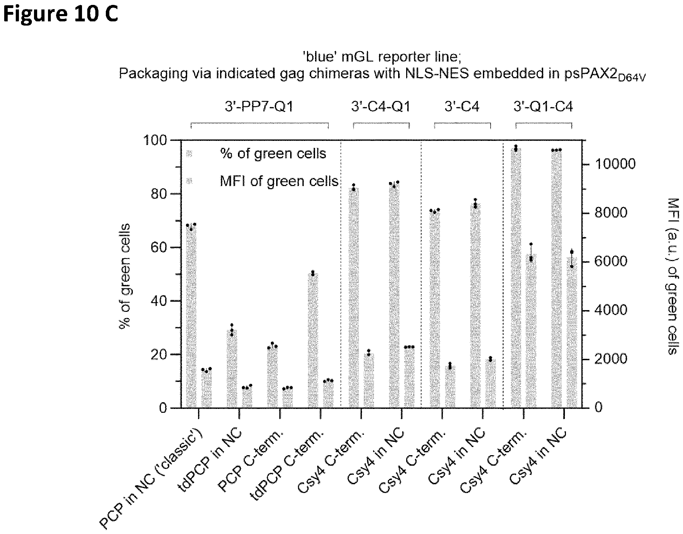

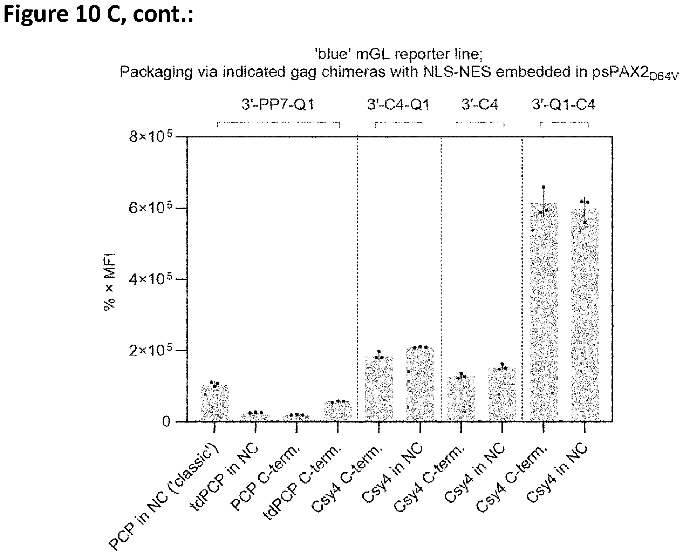

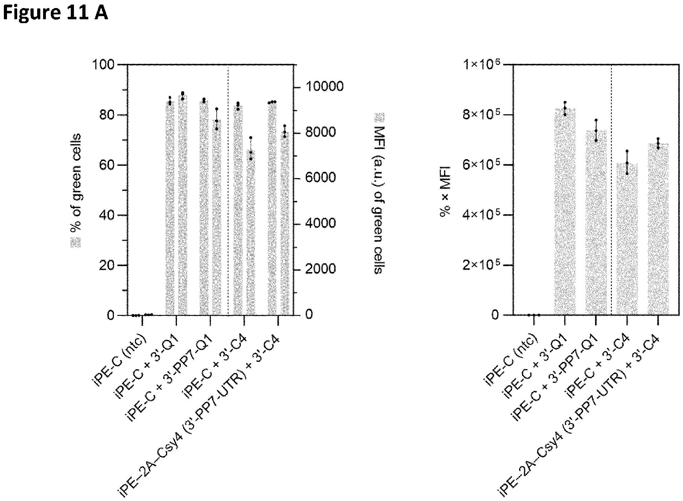

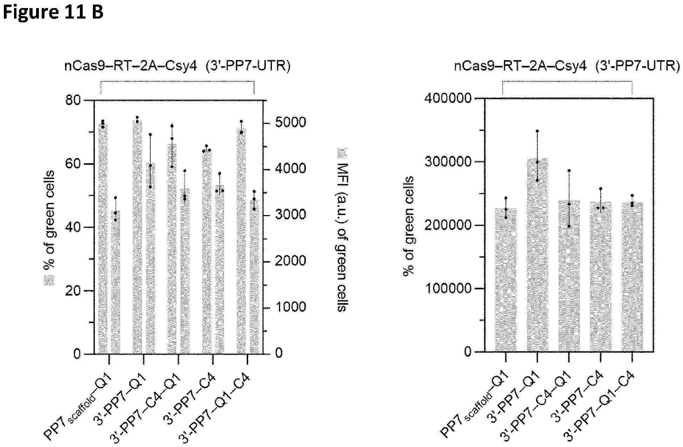

For further details, see also Figures 10 and 11.

The inventors surprisingly found that Csy4/C4 is not equivalent to other aptamer systems. So far, Csy4 has been used only biotechnologically for its processing activity to process fusion arrays of gRNAs and crRNAs to single components. The inventors are the first ones to show that Csy4 can be used for VLP-mediated export as well as protection of pegRNAs at the same time since pegRNA stability is a major bottleneck. Importantly, pegRNAs have to remain stable for a very long time, from producer cell line to VLPs, in the recipient cells all across the cytosol into the nucleus until it reaches its target.

Alternatively, instead of the Csy4/C4 system, the Rotavirus N-terminal fragment (aa 1-162) of the NSP3 protein comprising an acid sequence according to SEQ ID NO: 323 could be used for protecting an engineered pegRNA that ends with a 3'-gacc. To exactly terminate at gacc, DNA coding for the pegRNA needs to have downstream of the gacc motif a 3'-tRNA or 3'-tRNA mimicking motif or a ribozyme motif, such as the genomic or anti-genomic Hepatitis D virus (HDV) ribozyme, encoded. The tRNA is processed by various enzymes, such as RNAse P, which cleaves upstream of the tRNA and thus, when placed downstream of the gacc motif, can be used to generate exact 3'-ends. The genomic or anti-genomic HDV ribozyme is used with the same intention to generate exact 3'-ends when placed downstream of gacc. In contrast to tRNA, ribozymes are autocatalytically and thus do not require any host processing enzymes.

In one embodiment, the multi-functional fusion protein of the present invention is based on group-specific antigen (gag), such as HIV-1 gag, or on gag-pol polyprotein.

Group-specific antigen, or gag, is the polyprotein that contains the core structural proteins of a retrovirus. It makes up all the structural units of viral conformation and provides supportive framework for mature virion. All orthoretroviral gag proteins are processed by the protease (PR or *pro*) into MA /matrix), CA (capsid), NC (nucleocapsid) parts, and sometimes more. If Gag fails to cleave into its subunits, virion fails to mature and remains uninfective.

Gag-Pol protein mediates, with Gag polyprotein, the essential events in virion assembly, including binding the plasma membrane, making the protein-protein interactions necessary to create spherical particles, recruiting the viral Env proteins, and packaging the genomic RNA via direct interactions with the RNA packaging sequence (Psi). Gag-Pol polyprotein may regulate its own translation, by the binding genomic RNA in the 5'-UTR. At low concentration, the polyprotein would promote translation, whereas at high concentration, the polyprotein would encapsidate genomic RNA and then shut off translation.

In preferred embodiments, the multi-functional fusion protein of the present invention based on (group-specific antigen) gag and/or gag-pol, which preferably comprises any one of the following structures:

| | |
|---|---|
| Gag | *N*-myr-[MA]-[CA]-p2-[NC]-p1-[p6] |
| Gag-pol _{D64V} | *N*-myr-[MA]-[CA]-p2-[NC]-p1-[p6*]-PR-RT-INT_{D64V} |
| Gag_{L21S} | *N*-myr-[MA_{L21S}]-[CA]-p2-[NC]-p1-[p6] |
| Gag _{ΔZF+L21S}-PCP | *N*-myr-[MA_{L21S}]-[CA]-p2-[NC_{ZF1mutΔZF2}-PCP]-p1-[p6] |
| Gag_{ΔZF+L21S/NLS-NES}-PCP | *N*-myr-[MA_{L21S}]-[CA]-p2-[NC_{ZF1mutΔZF2+NLS-NES}-PCP]-p1-[p6] |

wherein
***N*-myr** refers to an N-terminal myristoyl;
**MA** refers to the matrix protein of Gag,
   such as p17 matrix protein of HIV-1;
**MA_{L21S}** refers to the matrix protein containing the mutation L21S;
**CA** refers to the capsid protein of Gag,
   such as p24 capsid protein of HIV-1;
**p1** refers to spacer peptide 1 of Gag;
**NC** refers to the nucleocapsid protein of Gag comprising zink finger motif 1 **(ZF1)** and zinc finger motif 2 **(ZF2)**
   such as p7 capsid protein of HIV-1,
**p2** refers to refers to spacer peptide 2 of Gag;
**p6** refers to the 6kDa polypeptide at the C-terminus of Gag;
**p6*** refers to the cryptic p6 peptide, which is expressed after ribosomal frame shifting;
**PR** refers to the protease of Gag;
**RT** refers to the reverse transcriptase of Gag;
**INT** refers to the integrase of Gag;
**PCP** refers to PP7 coat protein;
**NC_{ΔZF}-PCP** refers to a nucleocapsid protein wherein ZF 1 was inactivated by mutation of 3 cysteines to serine and wherein ZF2 was replaced by PCP;
**NC_{ΔZF+NLS-NES}-PCP** refers to a nucleocapsid protein wherein ZF 1 was inactivated by mutation of 3 cysteines to serine and wherein ZF2 was replaced by NLS-NES-PCP;
**NLS-NES** refers to s shuttling motif containing both a nuclear localization signal and a nuclear export signal,
   preferably NLS-NES-NLS_{FLAG}, wherein NLS_{FLAG} refers to a synthetic NLS such as NLS_{c-Myc}-NES_{HIV-1}-NLS_{FLAG};

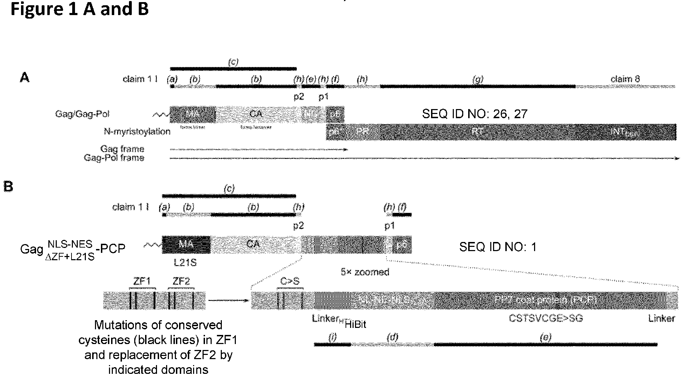

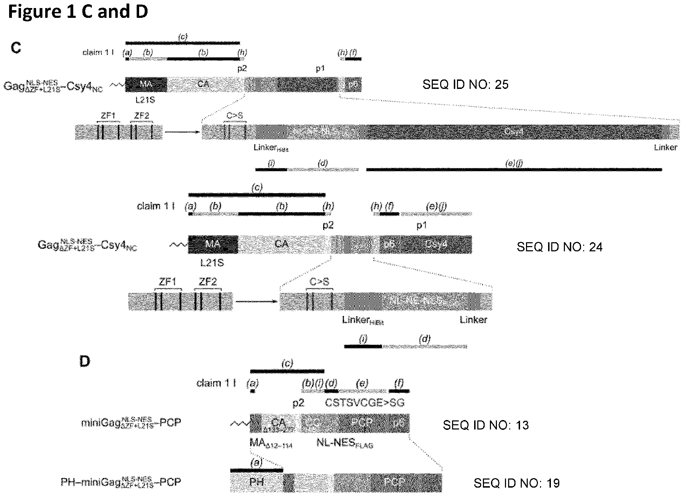

For further details, see also Figures 1, 2A, 3A.

In one embodiment, the multi-functional fusion protein of the present invention is based on truncated gag and/or on truncated gag-pol polyprotein.

In preferred embodiments, the multi-functional fusion protein of the present invention based on truncated gag, non-gag variants, and/or gag-pol is truncated in MA and CA and the NC domain is replaced by a shuttling motif and PCP, more preferably comprising any one of the following structures:

| | |
|---|---|
| miniGag_{ΔZF+L21S/NLS-NES}-PCP | *N*-myr-[MA_{Δ12-114}]-[CA_{Δ133-277}]-p2-CC-NLS-NES-PCP-[p6] |
| PHminiGag_{ΔZF+L21S/NLS-NES}-PCP | PH-[MA_{Δ12-114}]-[CA_{Δ133-277}]-p2-CC-NLS-NES-PCP-[p6] |

wherein
***N*-myr** refers to an N-terminal myristoyl;
**PH** refers to a pleckstrin homology domain;
**MA_{Δ12-114}** refers to the matrix protein of Gag wherein amino acid residues 12 to 114 are deleted;
**CA_{Δ133-277}** refers to the capsid protein of Gag wherein amino acid residues 133 to 277 are deleted;
**p2** refers to refers to spacer peptide 2 of Gag;
**CC** refers to a dimerization domain, preferably the general control transcription factor GCN4
**NLS-NES** refers to s shuttling motif containing both a nuclear localization signal and a nuclear export signal,
   preferably NLS-NES-NLS_{FLAG}, wherein NLS_{FLAG} refers to a synthetic NLS such as NLS_{c-Myc}-NES_{HIV-1}-NLS_{FLAG};
**PCP** refers to PP7 coat protein;
**p6** refers to the 6kDa polypeptide at the C-terminus of Gag;

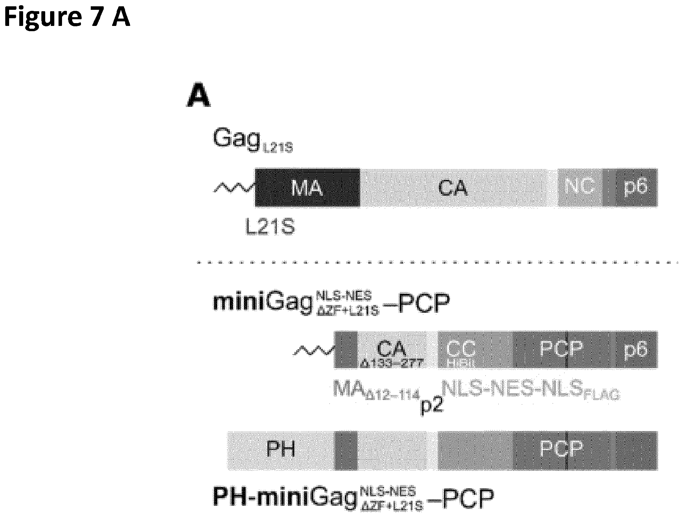

For further details, see also Figure 7A.

In preferred embodiments, the multi-functional fusion protein (I) of the present invention is based on group-specific antigen (gag), more preferably HIV-1 gag, and/or a gag-pol polyprotein, or a mini-Gag variant, and comprises an amino acid sequence selected from any one of SEQ ID NOs: 1 to 81. In preferred embodiments, the multi-functional protein (I) comprises an amino acid sequence selected from any one of SEQ ID NOs: 1, 2, 13, 19, 24, 25, 26, 27, 60, and 61.

In a preferred embodiment, the multi-functional fusion protein of the present invention comprises an amino acid sequence according to SEQ ID No: 60, and the following structure: *N*-myr-[MA]-[CA]-p2-[NC]-p1-[p6].

In a preferred embodiment, the multi-functional fusion protein of the present invention comprises an amino acid sequence according to SEQ ID No: 26 or 27, and the following structure:
*N*-myr-[MA]-[CA]-p2-[NC]-p1-[p6*]-PR-RT-INT_{D64V}.

In a preferred embodiment, the multi-functional fusion protein of the present invention comprises an amino acid sequence according to SEQ ID No: 61, and the following structure: *N*-myr-[MA_{L21S}]-[CA]-p2-[NC]-p1-[p6].

In a preferred embodiment, the multi-functional fusion protein of the present invention comprises an amino acid sequence according to SEQ ID No: 1, and the following structure: *N*-myr-[MA_{L21S}]-[CA]-p2-[NC_{ZF1mutΔZF2}-PCP]-p1-[p6].

In a preferred embodiment, the multi-functional fusion protein of the present invention comprises an amino acid sequence according to SEQ ID No: 2, and the following structure: *N*-myr-[MA_{L21S}]-[CA]-p2-[NC_{ZF1mutΔZF2+NLS-NES}-PCP]-p1-[p6].

In a preferred embodiment, the multi-functional fusion protein of the present invention comprises an amino acid sequence according to SEQ ID No: 13, and the following structure: *N*-myr-[MA_{Δ12-114}]-[CA_{Δ133-277}]-p2-CC-NLS-NES-PCP-[p6].

In a preferred embodiment, the multi-functional fusion protein of the present invention comprises an amino acid sequence according to SEQ ID No: 19, and the following structure: PH-[MA_{Δ12-114}]-[CA_{Δ133-277}]-p2-CC-NLS-NES-PCP-[p6].

The multi-functional fusion protein of the present invention exhibits a highly modular functionality which is achieved by one single protein, such as based on Gag, which comprises all of the following 8 functionalities:
(1) budding off of the membrane with encapsulated cargo,
(2) shuttling between nucleus and cytosol via optimized combined NLS/NES motifs to pick up nuclear cargo and transport to the budding site,
(3) binding and transport of specific RNA molecules, including mRNA, sgRNA, pegRNA, and optionally RNA for reverse transcription, tagged with specific RNA aptamer motifs,
(4) simultaneous co-packaging and delivery of protein components, such as modular gene editors, *e.g.,* Cas9-fusions to base editors or reverse transcriptase without the need for further modification (e.g., fusion or proteolytic activation) of the gene editor proteins,
(5) cellular uptake into the target cell via modular glycoproteins,
(6) delivery of the RNP into the nucleus of the target cell,
(7) optionally maturation of VLP for cellular uptake and delivery of RNAs, or RNPs or HDR-donor DNA,
(8) optional reverse transcriptase (RT) functionality for synthesizing DNA donors for HDR-mediated insertions, wherein the HDR donor-templates can be bound as RNA via one of the nucleic acid-binding domains, preferably NC or PCP, before reverse transcription in the VLP or the target cell.

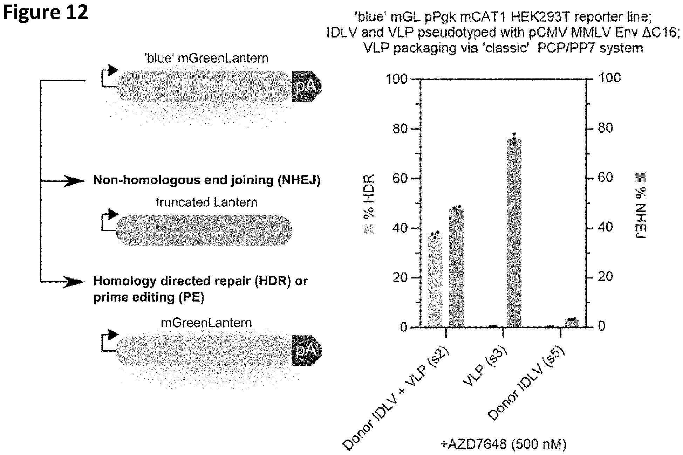

For further details, see also Figure 12.

In one embodiment, the HDR donor-templates can be bound as RNA via one of the nucleic acid-binding domains, preferably NC or PCP before reverse transcription in the VLP or the target cell.

As discussed above, the present invention provides a polynucleotide encoding the multi-functional fusion protein of the present invention.

The polynucleotide encoding the multi-functional fusion protein of the present invention preferably comprises or consists of a nucleotide sequence selected from SEQ ID NOs: 82 to 159.

As discussed above, the present invention provides the use of the multi-functional fusion protein of the present invention and/or the polynucleotide encoding it for generating a nucleic acid delivery system, preferably a virus-like particle (VLP).

As discussed above, the present invention provides the use of the multi-functional fusion protein of the present invention and/or the polynucleotide encoding it for delivering nucleic acids, preferably gene-editing nucleic acids, epigenome modulator nucleic acids, transcriptome-editing nucleic acids, and/or epitranscriptome modulator nucleic acids.

### Complex of the fusion protein with RNPs

As discussed above, the present invention provides a complex of at least one multi-functional fusion protein of the present invention with a gene-editing ribonucleoprotein (RNP) complex comprising an mRNA or a guide RNA.

A "guide RNA" is an RNA that recognizes a region of interest of RNA or DNA by binding to it. It forms stable complexes with a genome editing protein. These gene-editing ribonucleoprotein complexes are used to introduce deletions, insertions or otherwise alterations of the targeted RNA or DNA. They occur naturally, serving important functions, but can also be designed to be used for targeted editing, such as with CRISPR-Cas9, CRISPR-Cas12, or on RNA targets via Cas13.

As discussed above, the gRNA is a short synthetic RNA composed of
a scaffold sequence necessary for Cas-binding and
a nucleotide spacer, which has a length of about 20 nucleotides, that defines the genomic target to be modified.

Thus, the genomic target of the Cas protein is defined by the target sequence present in the gRNA.

Preferably, the guide RNA is a sgRNA, pegRNA, or Cas13.

In type II CRISPR/Cas system, single guide RNA (sgRNA) directs the target specific regions. Single guide RNAs result from the combination of two RNA molecules, the trans-activating RNA (tracrRNA) is responsible for Cas9 endonuclease activity while the CRISPR-RNA (crRNA) binds specifically to the DNA target region. Therefore, tracrRNA and crRNA are two key components and are joined at the tetraloop which results in the formation of sgRNA. The Cas9 endonuclease binds to the stem loop structure of the sgRNAThe spacer region usually consists of 18-20 base pairs and identifies the specific complementary target region, which is cleaved by Cas9 after its binding.

A "gRNA" as used herein refers to a single stranded RNA with nucleotide spacer and a scaffold, which contains a nucleotide nucleic acid binding domain (preferably PP7 or the Csy4 motif "C4") within an exposed loop or at the 3' end of the RNA.

A prime editing guide RNA ("pegRNA") as used herein refers to a prime editing gRNA with extended 3' end including a primer binding sequence (PBS) and a template for reverse transcription containing the desired RNA sequence. It directs the prime editor protein to the target locus and also encodes the desired edit.

Structured RNA motifs, such as tEvopreQ1, added to the 3' terminus of pegRNAs can enhance their stability and prevent degradation of the 3' extension.

Ribonucleoproteins (RNP) include a family of proteins present in the nucleus and cytoplasm that bind RNA to guide to a target nucleic acid and introduce a double strand break (DSB)

The protein(s) that is/are part of the RNP comprise(s) gene-editing polypeptide(s) or protein(s).

The "nucleic acid editor ribonucleoprotein complex", as used herein, including proteins from the CRISPR-Cas family, TALEs, pumilios, and de novo proteins, such as *de novo* DNA/RNA binding proteins, preferably a Cas protein such as Cas9 or Cas13 bound to the multifunctional protein via its aptamer-tagged gRNA component such that binding during packaging and unbinding in the target cell is independent of the modifications of the protein component, e.g. with base editors, reverse transcriptase, epigenome or epitranscriptome editors.

In a preferred embodiment, the gene-editing polypeptide(s) or protein(s) is/are CRISPR/Cas9 effectors.

In one embodiment, the CRISPR/Cas9 effectors comprise:
- a CRISPR-associated endonuclease (Cas protein or Cas9 nuclease),
- a reverse transcriptase domain, such as the MLV RT,
- Base editor domains, such as TadA, and/or
- Transactivating/repressing domains, such as VPR or KRAB.

In a preferred embodiment, the gene-editing polypeptide(s) or protein(s) comprise/s:
a. double-strand break generation activity,
b. nicking activity, or
c. nuclease-dead binding activity towards target nucleic acids, and
d. optionally a fusion with a nuclear localization signal (NLS) and/or nuclear export signal (NES), and
e. optionally, a fusion towards an effector domain towards an RNA- or DNA-dependent DNA polymerase activity, a base editor including but not limited to adenine and cytidine deaminases, optionally additionally fused to one or more copies of uracil glycosylase inhibitors (UGI), a transcriptional activator or repressor, an epigenetic modifier including DNA- and histone-(de)methylases or (de)acetylases, generic DNA-nicking domains, including but not limited to FokI, and recruiters thereof.

In an embodiment, where the complex comprises a complex of at least one multi-functional fusion protein of the present invention with a gene-editing ribonucleoprotein (RNP) complex comprising an mRNA, the mRNA preferably comprises an aptamer-tag.

### Nucleic acid delivery system

As discussed above, the present invention provides a nucleic acid delivery system.

The nucleic acid delivery system of the present invention comprises:
*(A)* at least one multi-functional fusion protein of the present invention;
*(B)* a gene-editing ribonucleoprotein (RNP) complex, which comprises
   *(B1)* an mRNA or a guide RNA, such as a sgRNA or pegRNA, and
   *(B2)* gene-editing polypeptide(s) or protein(s),
*(C)* a fusogenic (glyco)protein or protein complex comprising at least one multi-functional protein or a set of at least two proteins, wherein the multifunctional domain or the set of at least two proteins comprise a receptor-targeting and a fusogenic domain to fuse the VLP to the target cell membrane,
*(D)* a targeting domain, such as a glycoprotein or engineered variants thereof,
*(E)* a moiety mediating endosomal escape, such as a glycoprotein or engineered variants thereof,
*(F)* optionally, a reporter-signal generating gene product, such as a luciferase or a fluorescent protein, and
(*G*) optionally, a nucleic acid-stabilizing protein, preferably Csy4.

The nucleic acid delivery system of the present invention is preferably a virus-like particle (VLP).

In one embodiment, the fusogenic (glyco)protein or protein complex according to (*C*) is an envelope protein, or an engineered variant thereof.

### - Multi-functional fusion protein(s) (A)

The nucleic acid delivery system of the present invention comprises at least one multi-functional fusion protein of the present invention.

In one embodiment, the nucleic acid-binding (NAB) domain is fused to Vicugna pacos (Alpaca)/Synthetic CANTDcb 1, which binds to the capsid of HIV-1 Gag. The NAB-CANTDcb 1 fusion comprises the shuttling motif and is co-expressed with Gag-Pol.

In a preferred embodiment, the nucleic acid delivery system comprises two multi-functional fusion proteins of the present invention, which preferably comprises an amino acid sequence selected from any one of SEQ ID NOs: 26, 27, 56, 57 and 66.

In one embodiment, the nucleic acid editor complex (II) comprises a guide RNA, preferably a sgRNA or a pegRNA, which comprises the nucleic acid motif, bound by NAB domain *(e)* of (I) and an apo-protein of the nucleic acid editor complex, wherein the guide RNA is preferentially selected from a nucleic acid sequence according to any one of SEQ ID NOs: 160 to 169, while the apo-protein is preferentially selected from the SEQ ID NOs: 28 to 35.

In one embodiment, the nucleic acid editor complex (II) is a gene-editing ribonucleoprotein (RNP) complex, which comprises gene-editing polypeptide(s) or protein(s),
preferably
CRISPR/Cas9 effectors, more preferably a CRISPR-associated endonuclease (Cas9 nuclease or Cas13),
TALEs,
pumilio proteins,
*de novo* DNA/RNA binding proteins,
or mutants thereof,

and wherein the gene-editing polypeptide(s) or protein(s) preferably further comprise a nuclear localization signal (NLS), preferably NLS of c-Myc protein (NLS_{c-Myc}), NLS of SV40 (NLS_{SV40}) or a synthetic NLS, such as attenuated variants of NLS_{c-Myc} and NLS_{SV40}, or other mono- or bipartite synthetic NLS, more preferably wherein the NLS comprises an amino acid sequence according to any one of SEQ ID NOs: 48 to 54,
or a nuclear export signal (NES), preferably the NES of HIV-1 (NES_{HIV-1}) or DBR1, more preferably a NES comprising an amino acid sequence according to SEQ ID NO: 65,
or combinations thereof.

### - mRNA or ribonucleoprotein (RNP) complex (B)

The nucleic acid delivery system of the present invention comprises a gene-editing ribonucleoprotein (RNP) complex, which comprises
*(B1)* an mRNA or a guide RNA, such as a sgRNA or pegRNA, and
*(B2)* gene-editing polypeptide(s) or protein(s),

### - Nucleic acid (B1)

In one embodiment, the nucleic acid is an mRNA.

In one embodiment, the nucleic acid is a guide RNA (gRNA), such as a sgRNA or a pegRNA, as discussed above.

The nucleotide sequence of the nucleic acid depends, for instance, on the intended base editing in target loci such as e.g. *B2M.*

As discussed above, the gRNA is a short synthetic RNA composed of
a scaffold sequence necessary for Cas-binding and
a nucleotide spacer or flexible guide region, which has a length of about 15 to 30 nucleotides, preferably of about 20 nucleotides, that defines the genomic target to be modified.

Thus, the genomic target of the Cas protein is defined by the target sequence present in the gRNA.

In one embodiment, the nucleotide spacer or flexible guide region is a CRISPR spacer.

In one embodiment, the gRNA is a pegRNA.

Said specific nucleic acid comprises an RNA-motif which can be bound by the nucleic acid-binding domain *(f)* of the multi-functional fusion protein(s) *(A).* Said RNA-motif is preferably an aptamer.

Preferred examples are
PP7 coat protein aptamer,
MS2 coat protein aptamer,
com operator (which is the structure of a com RNA hairpin),
BoxB aptamer system,
P22,
Csy4 aptamer, and
HIV-1psi.

The RNA-motif is preferably the same as the RNA-motif of the gene editing ribonucleoprotein (RNP).

### - Gene editing component(s) (B2)

The nucleic acid delivery system of the present invention comprises a gene-editing components, in particular gene-editing polypeptide(s) or protein(s) *(B2).*

As discussed above, the gene-editing polypeptide(s) or protein(s) is/are preferably CRISPR/Cas9 effectors.

In one embodiment, the CRISPR/Cas9 effectors comprise:
- a CRISPR-associated endonuclease (Cas protein or Cas9 nuclease),
- a reverse transcriptase domain, such as MLV-RT,
- a base editor domain, such as TadA, and/or
- an epigenetic modifier domain, such as VPR.

In a preferred embodiment, the gene-editing polypeptide(s) or protein(s) comprise/s:
a. double-strand break generation activity,
b. nicking activity, or
c. nuclease-dead binding activity towards target nucleic acids, and
d. optionally a fusion with a nuclear localization signal (NLS) and/or nuclear export signal (NES), and
e. optionally, a fusion towards an effector domain towards an RNA- or DNA-dependent DNA polymerase activity, a base editor including but not limited to adenine and cytidine deaminases, optionally additionally fused to one or more copies of uracil glycosylase inhibitors (UGI), a transcriptional activator or repressor, an epigenetic modifier including DNA- and histone-(de)methylases or (de)acetylases, generic DNA-nicking domains including but not limited to FokI, and recruiters thereof.

The gene-editing component preferably contains an RNA-motif which can be bound by the nucleic acid-binding domain *(f)* of the multi-functional fusion protein(s) *(A).* Said RNA-motif is preferably an aptamer.

Preferred examples are:
PP7 coat protein aptamer,
MS2 coat protein aptamer,
com operator (which is the structure of a com RNA hairpin),
BoxB aptamer system,
P22,
Csy4 aptamer.

The RNA-motif is preferably the same as the RNA motif of the specific nucleic acid *(B1).*

### - Viral envelope protein (C)

The nucleic acid delivery system of the present invention comprises a viral envelope protein or viral envelope glycoprotein *(C).*

In one embodiment, the nucleic acid delivery system of the present invention comprises engineered variants of said viral envelope protein.

In one embodiment, the viral envelope protein is Vesicular stomatitis virus G (VSV G) protein.

In one embodiment, the viral envelope protein is a variant of VSV-G with a mutated target recognition domain and an additional fusion to a receptor-binding moiety, such as nanobodies or anticalines.

### - Targeting domain

The nucleic acid delivery system of the present invention comprises a targeting domain *(D).*

In one embodiment, the targeting domain *(D)* is a glycoprotein.

In one embodiment, the targeting domain *(D)* is an engineered variant of said glycoprotein.

Examples for a glycoprotein are VSV-G, MMLV-Env, FMLV-Env, TPMV H protein, SARS-CoV2 spike protein, Nipah virus G protein.

### - Moiety mediating endosomal escape (E)

The nucleic acid delivery system of the present invention comprises a moiety mediating endosomal escape *(E).*

In one embodiment, the moiety *(E)* is a glycoprotein.

In one embodiment, the moiety *(E)* is an engineered variant of said glycoprotein.

Examples for a glycoprotein are VSV-G, MMLV-Env, FMI,V-Env, TPMV F protein, SARS-CoV2 spike protein, Nipah virus G protein.

### - Reporter gene product (F)

nucleic acid delivery system of the present invention optionally comprises a reporter gene product *(F).*

In one embodiment, the moiety *(F)* is a luciferase or a fluorescent protein.

The inventors disclose the development of a novel virus-like particle (VLP) system that offers significant advantages over existing delivery methods.

In recent years, several VLP technologies with both lentiviral and retroviral origin have been developed for the delivery of genome editing systems. However, the VLP system disclosed herein has been optimized to achieve superior efficiency and effectiveness compared to existing systems. The VLP system of this invention is preferably based on a multi-functional fusion protein, preferably an engineered Gag protein fusion with an aptamer-binding protein, specifically the PP7 coat protein (PCP), which can bind to a PP7 RNA aptamer or the Csy4 protein binding to the C4 aptamer. To ensure efficient packaging of PP7-tagged sgRNAs or pegRNAs, the multi-functional fusion protein (such as the Gag-PCP fusion protein) also contains a nuclear localization signal (NLS) and a nuclear export signal (NES). This dual-signal strategy enables the multi-functional fusion protein to shuttle between the nucleus and the cytoplasm, ultimately ensuring efficient packaging and delivery of the genome editing complexes in the form of ribonucleoproteins (RNPs).

Many recent VLP designs based on the retro-/lentiviral gag polyprotein rely on the N-terminal fusion of a gene editing effector to the C-terminus of gag, utilizing an additional protease cleavage site for RNP release (Banskota *et al.,* 2022, Hamilton *et al.,* 2021, Mangeot *et al.* 2019, Haldrup *et al.* 2023), which can be transported into the nucleus of the receiver cell. While this is a very natural design for VLP-based protein delivery which even tackles the bottlenecks of cargo release and nuclear trafficking, it does not prevent the delivery of unloaded proteins which could reduce its effectiveness. Also, switching to other cargo proteins can impact packaging efficiency and might require re-optimization.

In contrast, the system of this invention disclosed herein directly captures the (pe)gRNA via the aptamer-binding protein making use of the inherent instability of (pe)gRNAs in the absence of Cas9, ensuring that only functional and active RNP complexes are packaged into the VLPs. With the added benefit of pseudotyping and tropism modulation, this innovative approach has the potential to greatly enhance the application of genome editing technologies in various fields of research and therapeutic development.

In one embodiment, the RNA species comprises an autonomous 3'-stabilization motif, preferably truncated evopreQ1 comprising a ribonucleic acid sequence according to SEQ ID NO: 171, and/or a motif comprising a ribonucleic acid sequence according to SEQ ID NO: 216 that is protected by an additional heterologously expressed nucleic acid-stabilizing domain (*j*) of (I), preferably C4/Csy4 comprising an amino acid sequence according to SEQ ID NO: 64, the N-terminal domain of rotavirus NSP3 comprising an acid sequence according to SEQ ID NO: 323, wherein the N-terminal domain of rotavirus NSP3 optionally protects 3'-gacc RNA ends, or combinations thereof.

### Polynucleotides and vectors

As discussed above, the present invention provides a plurality of nucleotides.

Said plurality of polynucleotides comprises:
*(1)* a first polynucleotide comprising a nucleic acid sequence encoding a multi-functional fusion protein of the present invention;
*(2)* a second polynucleotide comprising a nucleic acid sequence encoding a gene editor protein;
*(3)* a third polynucleotide comprising a nucleic acid sequence encoding a guide RNA, such as a sgRNA or pegRNA, which is bound via the nucleic acid-binding domain (e), preferably as ribonucleoprotein complex; and
*(4)* a fourth polynucleotide comprising a nucleic acid sequence encoding a viral envelope protein.

As discussed above, the present invention provides one or more vectors comprising the plurality of polynucleotides of the present invention.

In one embodiment, the polynucleotide encoding the multi-functional protein (I) comprises a nucleotide sequence selected from any one of SEQ ID NOs: 82 to 106.

In one embodiment, the polynucleotide encoding the multi-functional protein (I) comprises a nucleotide sequence selected from any one of the SEQ ID NOs. as depicted below:

| | SEQ ID NO: |
|---|---|
| gag-L21S(ZF1(C>S)-ΔZF2):HiBiT:NLS-NES-NLS:PCP | 82 |
| gag-L21S(ZF1(C>S)-ΔZF2):HiBiT:PCP | 83 |
| gag(ZF1(C>S)-ΔZF2):HiBiT:NLS-NES-NLS:PCP | 84 |
| gag(ZF1(C>S)-ΔZF2):HiBiT:PCP | 85 |
| PLC-PHP:Gag-L21S(ZF1(C>S)-ΔZF2)::HiBit-PCP | 88 |
| PLC-PHP:Gag-S21L-mut-reversion(ZF1(C>S)-ΔZF2)::HiBit-PCP | 89 |
| PLC-PHP:Gag-L21S-NC(HiBit-PCP) | 91 |
| PLC-PHP:Gag-L21S(ZF1(C>S)-ΔZF2)::HiBit:NLS-NES-NLS TCP | 92 |
| minimal-gag(new-codons)_(Δ12-114_Δ133-277_ΔNC-p1)_GCN4:HiBit:PCP:p6 | 93 |
| minimal-gag(new-codons)_(Δ12-114_Δ133-277_ΔNC-p1)_GCN4:HiBit:NLS-NES-NLS:PCP:p6 | 94 |
| minimal-gag(new-codons)_(Δ12-114_Δ133-277_ΔNC-p1)_GCN4:HiBit:KKKKNG-NLS-NES:PCP:p6 | 95 |
| minimal-gag(new-codons)_(Δ12-114_Δ133-277_ΔNC-p1)_GCN4:HiBit:SV40-NLS-NES:PCP:p6 | 96 |
| minimal-gag(new-codons)_(Δ12-114_ΔNC-p1)_GCN4:HiBit:PCP:p6 | 97 |
| minimal-gag(new-codons)_(Δ12-114_ΔNC-p1)_GCN4:HiBit:NLS-NES-NLS:PCP:p6 | 98 |
| PLC-PHP:minimal-gag(new-codons)_(Δ12-114_Δ133-277_ΔNC-p1)_GCN4:HiBit:PCP:p6 | 99 |
| PLC-PHP:minimal-gag(new-codons)_(Δ12-114_Δ133-277_ΔNC-p1)_GCN4:HiBit:NLS-NES-NLS:PCP:p6 | 100 |
| PLC-PHP:minimal-gag(new-codons)_(Δ12-114_Δ133-277_ΔNC-p1)_GCN4:HiBit:KKKKLD-NLS-NES:PCP:p6 | 101 |
| PLC-PHP:minimal-gag(new-codons)_(Δ12-114_Δ133-277_ΔNC-p1)_GCN4:HiBit:c-Myc_NLS-NES:PCP:p6 | 102 |
| PLC-PHP:minimal-gag(new-codons)_(Δ12-114_Δ133-277_ΔNC-p1)_GCN4:HiBit:KKKKNG-NLS-NES:PCP:p6 | 103 |
| PLC-PHP:minimal-gag(new-codons)_(Δ12-114_Δ133-277_ΔNC-p1)_GCN4:HiBit:SV40-NLS-NES:PCP:p6 | 104 |
| gag-L21S(ZF1(C>S)-ΔZF2)-HiBiT-NLS-NES-NLS-p1-p6-Csy4 | 105 |
| gag-L21S(ZF1(C>S)-ΔZF2):HiBiT:NLS-NES-NLS:Csy4 | 106 |

In one embodiment, the present invention relates to three vectors or plasmids.

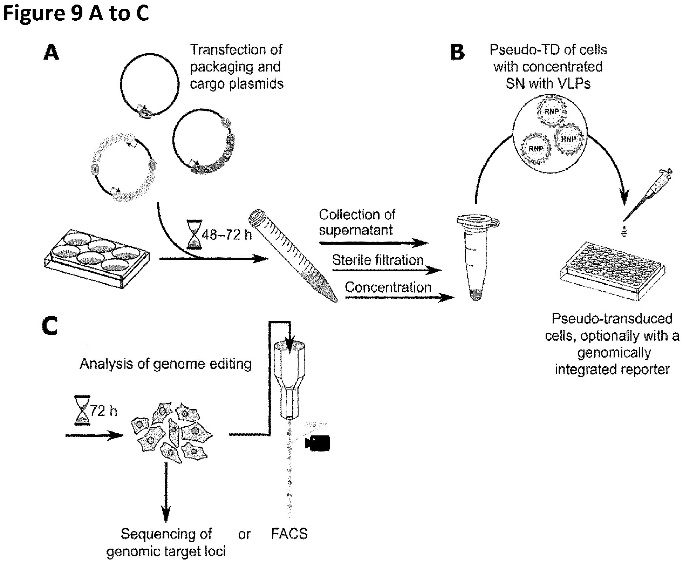

As an example, see Figure 9.

A further aspect of this invention relates to a set of polynucleotides encoding the set of at least two proteins (I),
preferably comprising the nucleotide sequence of SEQ ID NO: 107 (psPAXD64V (Gag-Pol) and a nucleotide sequence selected from any one of SEQ ID NOs. 82 to 106.

### Pharmaceutical compositions

As discussed above, the present invention provides a pharmaceutical composition.

A pharmaceutical composition of the present invention comprises:
the polynucleotide of the present invention which encodes the fusion protein of the present invention, the nucleic acid delivery system of the present invention, or the plurality of polynucleotides of the present invention;
optionally, pharmaceutically acceptable excipients and/or carrier.

### Producer cells, cell lines and kits

As discussed above, the present invention provides a cell or cell line.

Said cell or cell line comprises the multi-functional fusion protein of the present invention, the polynucleotide of the present invention, the nucleic acid delivery system of the present invention, the plurality of polynucleotides of the present invention.

The cell or cell line is preferably a mammalian cell or cell line, more preferably a human cell or cell line. The cell or cell line preferably has high transfectability, including but not limited to HEK293T cells and other human immortalized cells/cell lines.

The cell or cell line of the present invention is preferably a producer cell or cell line, which produces the nucleic acid delivery system of the present invention, preferably a VLP.

In one embodiment, the VLP will be in the supernatant of such a producer cell/cell line.

In one embodiment, the cell or cell line can be co-treated with exogenous compounds, such as by means of lipofection, electroporation, photoporation, sonoporation, particle bombardment, microinjection, magnetofection, viral transduction, cell-penetrating peptides, calcium-phosphate, nanoparticles, or combinations thereof, wherein the exogenous compounds are preferably selected from synthetic nanoparticles, fluorophores, small pharmaceutical compounds, positron emission tomography (PET) tracers, peptides, proteins, lipids, co-factors, small biomolecules, and combinations thereof.

In one embodiment, the exogenous compounds are compounds are not produced by the packaging cell line. In one embodiment, the exogenous compounds are imported for co-packaging into the VLPs. In one embodiment, the packaging cell line (to a lesser degree a cell line implanted in vivo) can be co-treated with exogenous compounds even if this is toxic to the packaging cell line as long as the VLP-product is as desired. In one embodiment, the system is a 'self-referential system'.

A further aspect of this invention relates to the use of a cell or cell line according to this invention for:
- *ex vivo* generating a therapeutic cell line, a diagnostic cell line, or a cell line for tissue engineering,
- generating a VLP-distributing cell line, which preferably can be implanted into a combination of cells, a tissue and/or organism to produce VLPs therein.

Importantly, the cell or cell line according to this invention can be used for generating cells, such as cells that can communicate with other cells to, *e.g.,* support diagnostic and/or therapeutic applications.

In one embodiment, the cell or cell line according to this invention can be used for tissue engineering and/or for preclinical applications.

As discussed above, the present invention provides kits.

A kit of the present invention comprises:
the polynucleotide of the present invention which encodes the fusion protein of the present invention, or
the nucleic acid delivery system of the present invention, or
the plurality of polynucleotides of the present invention, or
the one or more vectors of the present invention, or
the cell or cell line of the present invention.

As discussed above, the present invention provides a nucleic acid delivery system, preferably a virus-like particle, produced by transfecting, transducing, electroporating, lipofecting, photoporating, sonoporating, particle bombardment, microinjecting, magnetofecting, or otherwise inserting the plurality of polynucleotides of the present invention or the one or more vectors of the present invention into a cell and expressing the components of the nucleic acid delivery system from the plurality of polynucleotides or the one or more vectors of the present invention in the cell, thereby allowing the nucleic acid delivery system, preferably the virus-like particle, to spontaneously assemble in the cell.

### Medical uses

As discussed above, the present invention provides the nucleic acid delivery system of the present invention for use in medicine.

As discussed above, the present invention provides the nucleic acid delivery system of the present invention for use in the treatment of genetic diseases.

In one embodiment, the genetic disease is a monogenetic disease, such as cystic fibrosis, sickle cell anemia or Huntington's disease.

In one embodiment, the genetic disease is a polygenetic disease, such as diabetes, coronary artery disease or Alzheimer's disease.

### Base editing methods

As discussed above, the present invention provides an *in vitro* or *in vivo* method of editing a nucleic acid molecule in a target cell.

In one embodiment, the *in vitro* or *in vivo* method of editing a nucleic acid molecule in a target cell occurs by nuclease mediated editing, base editing or prime editing, or homology directed repair, such as by co-delivery of an integrase-deficient (D64V) lentivirus (IDLV), wherein, preferably, the IDLV is packaged using a psi-NC interaction, a PP7-PCP, MS2-MCP, and/or a C4-Csy4 interaction. In one embodiment, the *in vitro* or *in vivo* method of editing a nucleic acid molecule in a target cell occurs by nuclease mediated editing, base editing or prime editing, or homology directed repair, such as by co-delivery of an integrase-deficient (D64V) lentivirus (IDLV), wherein, preferably, the IDLV is packaged using a psi-NC interaction, a PP7-PCP, MS2-MCP, and/or a C4-Csy4 interaction, and the method comprises the step of delivering Cas9.

Said method comprises:
contacting the target cell with a nucleic acid delivery system of the present invention, or with the pharmaceutical composition of the present invention,
thereby installing one or more modifications to the nucleic acid at a target site.

Preferably, the target cell is a mammalian cell, more preferably a human cell.

In one embodiment, the target cell is in a subject, preferably a mammal, more preferably a human.

Preferably, the one or more modifications to the nucleic acid are associated with reducing, relieving or preventing symptoms of a disease or disorder.

### Method for nucleic acid delivery

As discussed above, the present invention provides a method for nucleic acid delivery, preferably gene-editing nucleic acids.

Said method comprises the steps of:
(I) providing a multi-functional fusion protein of the present invention to a cell,
   wherein said cell produces in its nucleus a gene-editing ribonucleoprotein (RNP) complex, which comprises a genome editor protein and a guide RNA, such as an aptamer-tagged sgRNA or pegRNA, wherein said gene-editing ribonucleoprotein (RNP) complex is as defined herein,
(II) allowing the multi-functional fusion protein to locate to the nucleus of said cell;
(III) forming a complex of the multi-functional fusion protein with the gene-editing ribonucleoprotein (RNP) complex in the nucleus;
(IV) allowing the complex of (III) to be exported to the cytosol of the cell;
(V) budding of virus-like particles comprising the gene-editing ribonucleoprotein (RNP) complex;
(VI) binding of the VLPs to a target cell surface,
   preferably via a specific receptor,
(VII) internalization of the VLPs into the target cell,
   preferably via endocytosis,
(VIII) transport of the internalized complex from (VII) into the cytosol,
   such as via endosomal escape,
(IX) dissociation of the RNP (with bound nucleic acid) or a nucleic acid from the nucleic acid binding domain of (I),
(X) optionally, transport of the RNP into the nucleus for genome editing or transactivation,
(XI) optionally, binding of the guide-RNA-editing complex to an RNA target sequence.

### Preferred embodiments

### - Abstract

Recent gene editing technologies, such as prime editors (PEs) and base editors (BEs), have shown great potential for the cure of genetic diseases. However, efficient and safe delivery of these genome editing complexes still poses a major challenge. After careful evaluation of existing delivery systems, the inventors developed a system for the transgene-free delivery of functional ribonucleoprotein (RNP) complexes that address current bottlenecks of existing systems such as efficient (pe)gRNA packaging. The highly optimized system comprises a chimeric fusion of Gag and the PP7 coat protein (PCP), which binds to a PP7 aptamer grafted into the (pe)gRNA scaffold, and thus allows for modular packaging of any RNP configuration. With the delivery system of the present invention, the inventors demonstrate the delivery of functional RNP complexes for prime editing and other CRISPR effectors in various cell types, including cortical neurons, T cells, and induced pluripotent stem cells. Finally, the inventors utilize a minimal-Gag variant with deletions in MA, CA, and NC, which shows comparable efficacies and is not reliant on the co-expression of HIV-1 wild-type gag-pol while offering improved efficacy and safety for delivery of genome editing complexes and thereby providing a valuable resource for laboratory implementation with additional potential to advance the somatic therapeutic application of gene editing technologies.

### - Results

### HIV-1-based VLPs for mRNA delivery.

Initial results with the eVLP system (Banskota *et al.,* 2022) showed highly effective delivery of base editors, but achieved modest targeting efficacy with an analogously designed eVLP architecture-based prime editor (PE). Motivated by this observation, the inventors sought to systematically decipher the bottlenecks of such a delivery system using the extensively studied HIV-1-based VLP system.

According to various studies (Wang *et al.,* 2019; Ma *et al.,* 2016; Hendel *et al.,* 2015), the half-lives of sgRNAs are magnitudes shorter compared to sgRNAs embedded in a ribonucleoprotein (RNP) complex, leading to the conclusion that especially pegRNAs, which carry a 3'-extended region encoding the desired edit and a primer binding site (PBS), are particularly prone to exonucleolytic degradation. The inventors reasoned that having the recruitment mechanism depend on the least stable component, that is the (pe)gRNA, of the complex would increase the likelihood of packaging functional RNPs exclusively.

The inventors based their system on the Gag protein of the Human Immunodeficiency Virus type 1 (HIV-1), which is a 55 kDa precursor responsible for assembly, budding, and maturation with the Gag-Pol protein possessing additional enzymatic functions, including reverse transcriptase and integrase activities, crucial for the virus lifecycle.

The inventors modified the first zinc finger motif within the NC domain by mutating all cysteine residues to serine to prevent unspecific RNA binding and replaced the second zinc finger motif by a non-oligomerizing mutant of the aptamer-binding domain PP7 coat protein (PCP) to bind and recruit RNA molecules tagged with the PP7 aptamer (Figure 1B). In addition, the inventors introduced L21S in the MA domain of Gag which has been shown previously to enhance the budding efficacy (Sherer *et al.,* 2009)). The inventors utilized this architecture previously to secrete barcoded introns out of a cell for non-invasive transcript quantification (Truong *et al.,* 2022) a concept that has recently gained strong interest (Horns *et al.,* 2023).

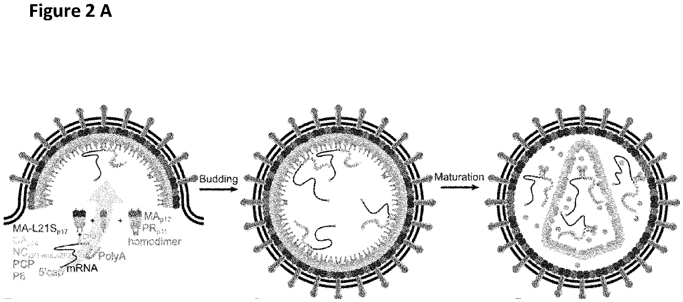

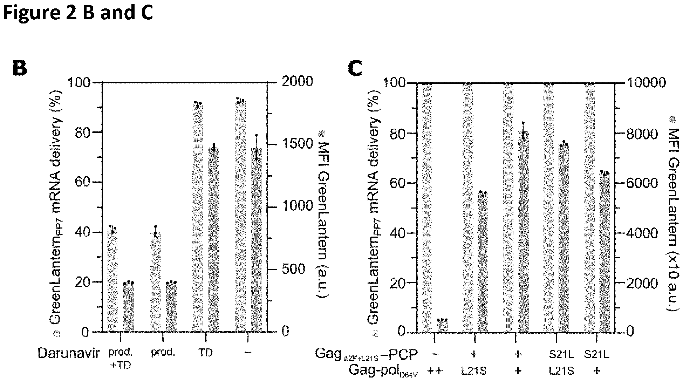

VLPs containing the mRNA information can be delivered to other cells when equipped with a suitable surface glycoprotein such as VSV-G (Lu *et al.,* 2019). The inventors thus co-expressed VSV-G and mGreenLantern (mGL) with a PP7 aptamer in its 3'UTR to determine essential elements required for efficient cargo packaging, budding, or target cell entry (Figure 2A). Since the proteolytic processing of VLPs is assumed to be as critical for VLP transduction efficacy as it is tightly regulated, the most efficient VLP delivery systems rely on mosaic setups with the protease activity provided by lentiviral Gag-pol plasmids like psPAX2 with an inactivated integrase domain (D64V). Consistent with these findings, the inventors also observed that the HIV-1 protease was essential for delivery of PP7-tagged mRNA coding for mGL (mGL_{PP7}), as the HIV-1 protease inhibitor darunavir significantly decreased delivery efficiency when supplied during production (Figure 2B).

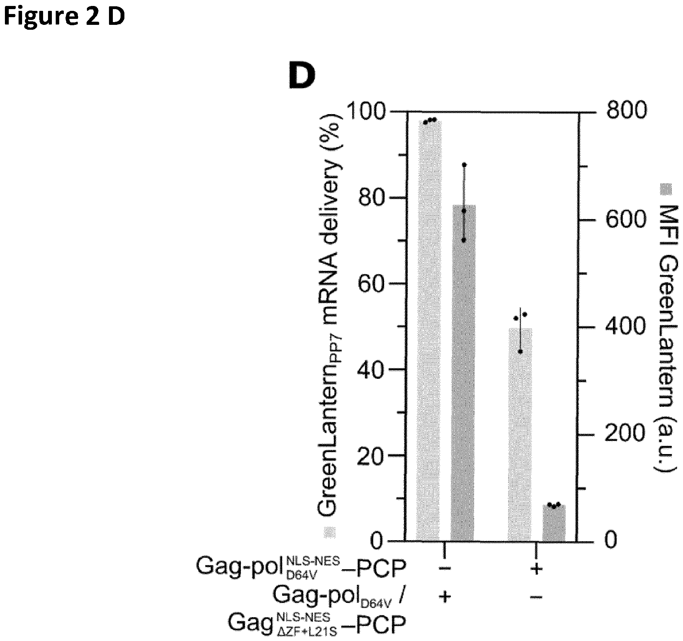

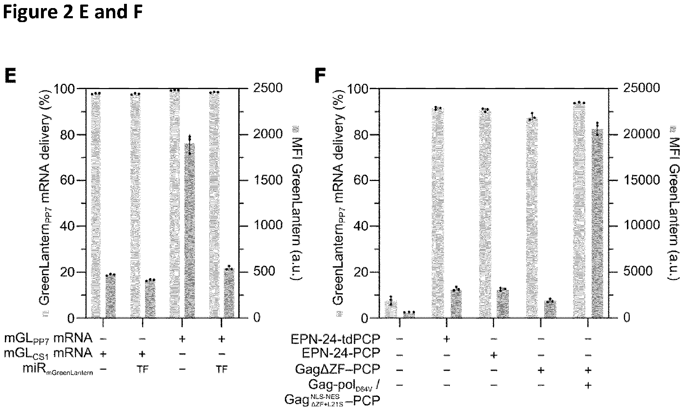

The inventors analyzed the impact of the L21S mutation, which is most beneficial when present in only half of the particle-forming on the Gag that has the PCP grafted into the ZF2 of the NC domain (Figure 2C); this combination is superior, especially compared to directly grafting PCP into the NC domain of Gag-Pol (Figure 2D). This was not surprising, since modifications of the NC domain, more specifically, the zinc finger motifs ZF1 and ZF2, often result in detrimental effects on the assembly of particles (Grigorov *et al.,* 2007, Boutant *et al.,* 2020). Thus, introduction of L21S likely compensates for the allosteric switch mediated naturally by the NC domain after binding to its genomic RNA. The inventors next set out to assess which fraction of green fluorescence of the transduced cells originated from mRNA delivery rather than protein delivery. Using the CS1 aptamer as a negative control for PCP-specific loading of mGL mRNA, the inventors found that still, almost all cells showed green fluorescence, even though the fluorescence intensity was significantly reduced. Even co-transfection of microRNA (miR) targeting mGL did not completely abolish fluorescence, indicating that a substantial amount of mGL protein was co-transduced into the target cells (Figure 2E).

To provide a calibration for the performance of the mosaic Gag-Pol:Gag-PCP system against recently published experiments with *de novo* protein cages, the inventors compared EPN-24-PCP nanocages with a C-terminal fusion of PCP or single-chain tandem-PCP and found its fluorescence intensity to be roughly on the same level as the non-chimeric Gag-PCP without Gag-Pol, but 8-fold lower than the co-expression of Gag-PCP and the integrase-deficient Gag-Pol (Figure 2F).

### Nuclear-cytosolic shuttling of Gag enables efficient delivery of various genome editors.

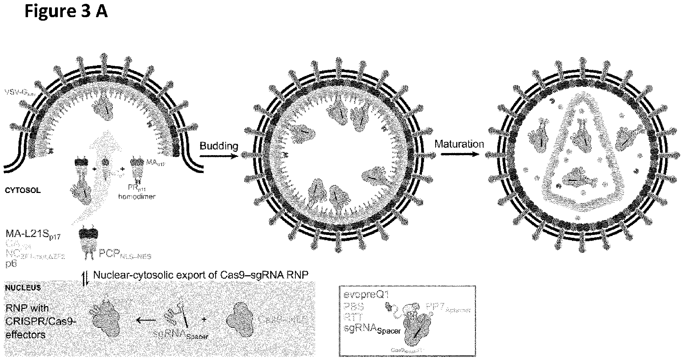

While Cas9 delivery encoded as mRNA that needs to be translated in the recipient cell is straightforward, delivering (pe)gRNAs is more challenging. Because (pe)gRNAs do not naturally reside in the cytosol, the inventors reasoned that the VLP system benefits from an active re-localization of (pe)gRNAs to the cytosol. The inventors consequently introduced a nuclear localization sequence (NLS) and a nuclear export sequence (NES) to Gag-PCP in order to shuttle (pe)gRNAs out of the nucleus (Figure 1B and 3A). For (pe)gRNA packaging, the inventors utilized a previous design from the inventors where the PP7 aptamer was grafted into the tetraloop (junction between tracrRNA and crRNA) of the SpCas9 gRNA/pegRNA scaffold (Figure 3A).

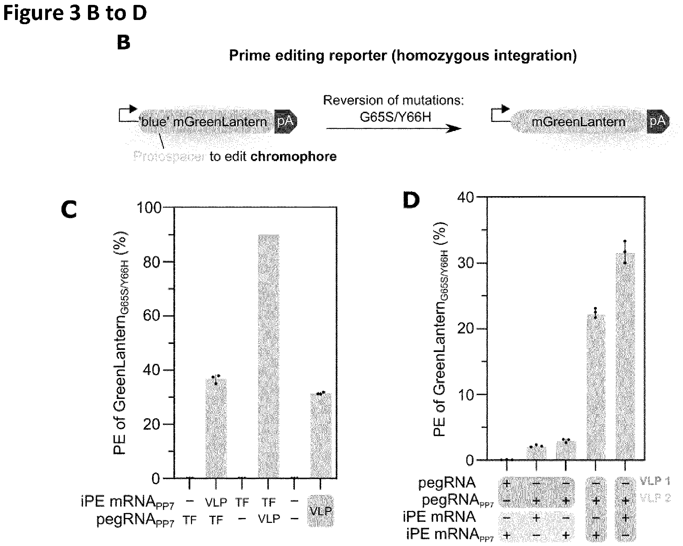

To test the VLP system for the delivery of functional prime editors, the inventors used a dedicated fluorescent reporter system in which mutations in the chromophore of mGreenLantern (G65S and Y66H) are corrected to shift the fluorescence profile from blue back to green (Figure 3B). Firstly, the inventors determined which individual component of the prime editing complex (pegRNA + prime editor) was limiting. To this end, the inventors provided one component via plasmid transfection while delivering the other component as PP7-tagged RNA via the VLP system of this invention. The results indicate that the pegRNA was delivered very effectively, while the delivery of prime editor (PE) mRNA was limiting, which lead to the assumption that packaging of the long mRNA encoding the prime editor might be the bottleneck (Figure 3C).

### Genome editors are delivered as pre-assembled ribonucleoproteins (RNPs).

Since any cargo mRNA is already translated in the VLP-producing cell, the inventors wanted to ascertain if the respective components were co-delivered as RNA molecules or as pre-assembled ribonucleoproteins (RNP). Others have demonstrated previously that aptamer-engrafted gRNAs can facilitate the recruitment of RNPs into VLPs (Lyu *et al.,* 2019). To this end, the inventors tested if the separate packaging of each component before co-transduction would abrogate the editing efficacy by preventing any RNP assembly in VLP-producing cells. The inventors observed a substantial reduction in editing events, which led the inventors to believe that the efficacy the inventors initially observed originated from RNP delivery. Interestingly, the PP7 tag had little to no influence on efficacy during separated packaging of the iPE mRNA, suggesting that the residual editing activity originates from unspecific packaging of either the mRNA or the iPE protein. The inventors proceeded to co-package the PP7-tagged pegRNA with the PP7-tagged iPE mRNA, achieving just above 50% editing in a HEK293T PE reporter cell line. The inventors validated the importance of all key components of the delivery system, particularly that of the NLS-NES module for (pe)gRNA shuttling, which resulted in a 6.4-fold increase in editing.

To determine any proportion of potential iPE mRNA delivery, the inventors transfected the reporter cell line with a microRNA (miR) targeting the 3' UTR of the iPE mRNA, prior to VLP delivery As the editing efficacy in the recipient cells was not reduced at all in the presence of miRs, the inventors concluded that editing events in the reporter cell line were exclusively mediated by RNP delivery and not by *de novo* translation of iPE mRNA and subsequent assembly with the co-delivered pegRNA. The same miR was pre-validated in an previous experiment, where it significantly reduced the translation of VLP-delivered mGreenLantern mRNA.

With the aim of developing a one-component packaging system, the inventors grafted the PP7-coat protein into the NC domain of Gag-pol and were able to deliver functional prime editing RNPs into target cells to mediate prime editing events, albeit with 3-fold reduced efficiency, when compared to the mosaic set-up using Gag-pol and Gag-PCP.

The inventors have thus established and validated a model for prime editor packaging and delivery improved by key modifications, where a PP7-tagged (pe)gRNA initially forms an RNP with a nucleus-localized genome editor protein to then be shuttled out of the nucleus by a modified VLP-forming gag unit. This system prevents the potential packaging of 'empty' prime editor proteins, while also providing higher stability for the gRNA prior to nuclear export.

### Optimization of the RNP delivery system.

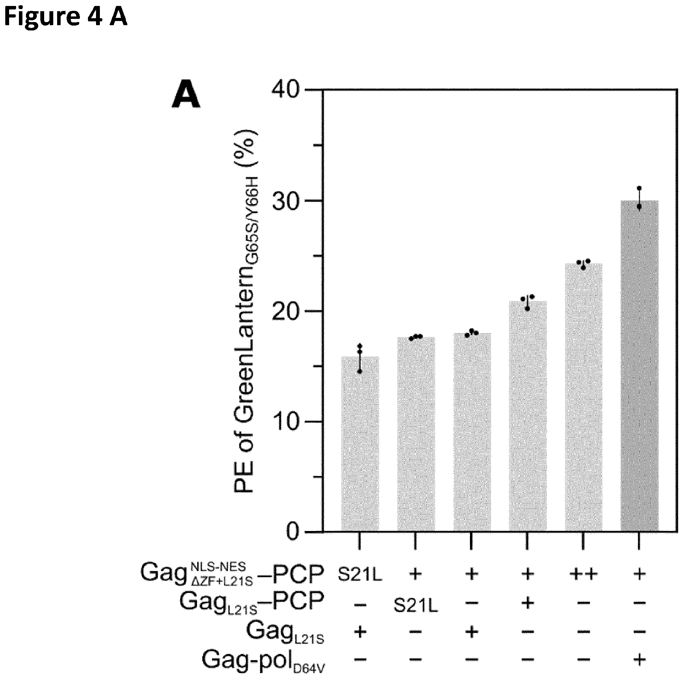

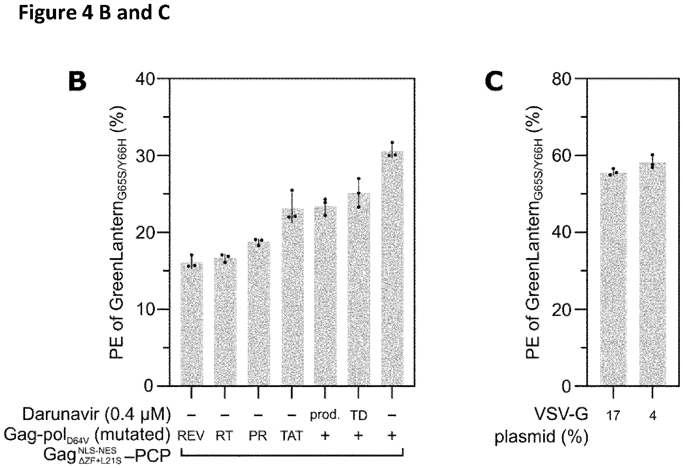

After establishing the system for delivery for genome editors, the inventors set out to optimize packaging stoichiometries as well as the Gag-PCP construct. Intriguingly, observations from mRNA delivery did not seamlessly translate to RNP delivery. For example, while the unsuccessful attempts to entirely omit Gag-pol2_{D64V} indicated that certain factors of gag-pol are crucial for efficient mRNA delivery, the cost of omitting Gag-pol_{D64V} was much smaller when delivering RNPs (Figures 2F and 4A). The inventors more closely investigated the components provided by Gag-pol_{D64V} and found small individual effects for each component. Intriguingly, the negative impact of protease inhibition or knockout on RNP delivery was quite small compared to mRNA delivery but also occurs with post-transduction treatment (Figures 2B and 4B).

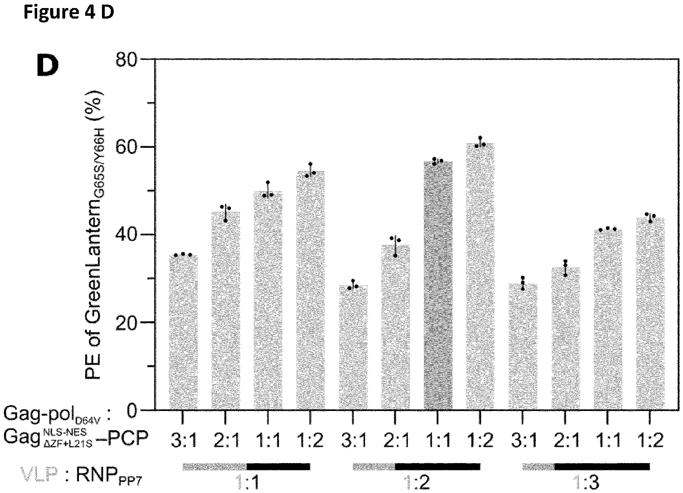

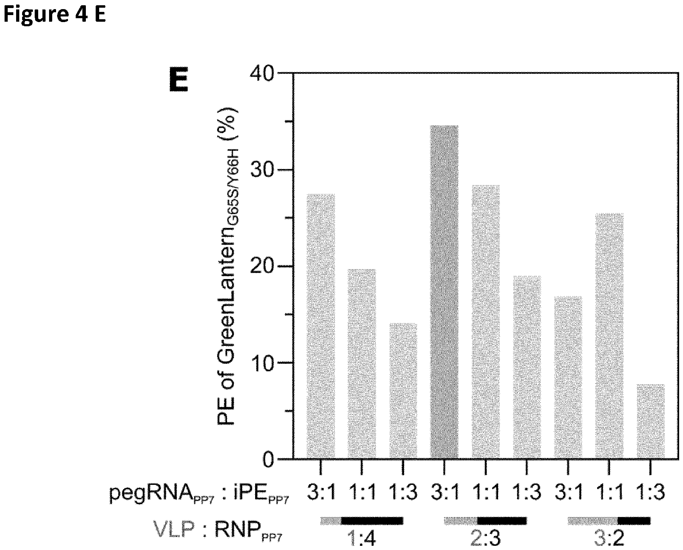

The inventors lastly optimized the amount of the VSV-G for pseudotyping (Figure 4C) as well as the stoichiometry of all delivery components (Figures 4D and 4E). The inventors specifically found that a slight excess (2: 1) of cargo over VLP gives optimal results. Regarding the proportion of each budding component, Gag-pol_{D64V} gave optimal results when supplied in slight deficit in relation to the inventors' engineered Gag. As a ratio of 1:1 was also close to optimal, the inventors decided to utilize this ratio to construct a combined plasmid where both components are expressed at identical strength (Gag-pol_{D64V}/Gag_{NLSNES_ΔZF2+L21S}-PCP). The optimal ratio for RNP formation appeared to be a 3-fold excess of pegRNA over the prime editor, which the inventors assumed to be transferrable to other Cas9 effector strategies as well.

### Application of the modular delivery system for delivery of base editors and other Cas9 effectors.

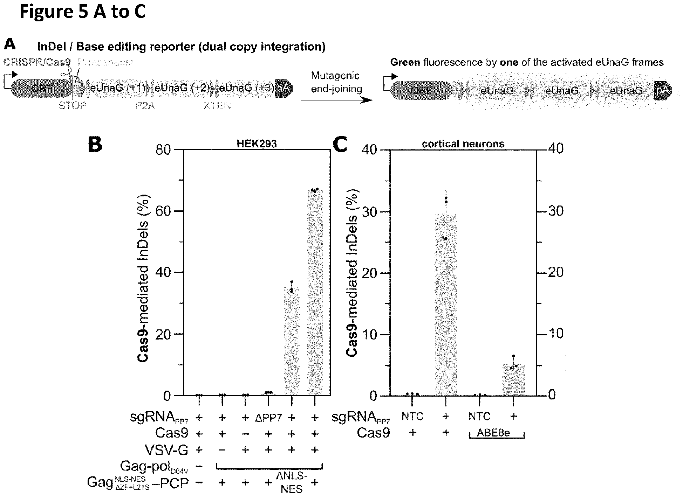

Since packaging occurs via the handle of the (pe)gRNA, the inventors naturally sought to generalize to the transduction of other Cas9 effectors, which is useable without any modifications. The inventors initially tested the delivery of active Cas9 and adenine base editors (ABEs) using a fluorescent reporter system, which can detect mutagenic end joining (mutEJ) events (InDels) as well as certain adenine base editing (BE) events. Specifically, when the stop codon is deleted, either via mutEJ or BE, one of the green fluorescence protein eUnaG in one of the three downstream frames will be activated (depending on InDel size) (Figure 5A). Upon RNP delivery to this stable reporter cell line, the inventors observed an InDel rate of above 60% in HEK293T cells and 12% in hiPSCs (Figure 5B). The inventors confirmed that as with prime editing, all components are essential to mediate any level of editing at all, and that the additional introduction of the shuttling motif increases the editing efficacy by up to 5-fold (Figure 5B).

The inventors next wanted to apply the delivery system of the present invention for base editing of a reference target site in the *B2M* locus, comparing the efficacy between various types of cell lines (Figure 5C). The inventors found that the present delivery system was able to facilitate the corresponding base edit to a high proportion in all tested cell lines, reaching an efficiency of 90% in cortical neurons and, at the lower end, still above 55% in hiPSCs. We, therefore, demonstrate the capability of mediating gene editing even in cell lines with potential clinical relevance, which are typically challenging to genetically modify using conventional techniques such as lipofection.

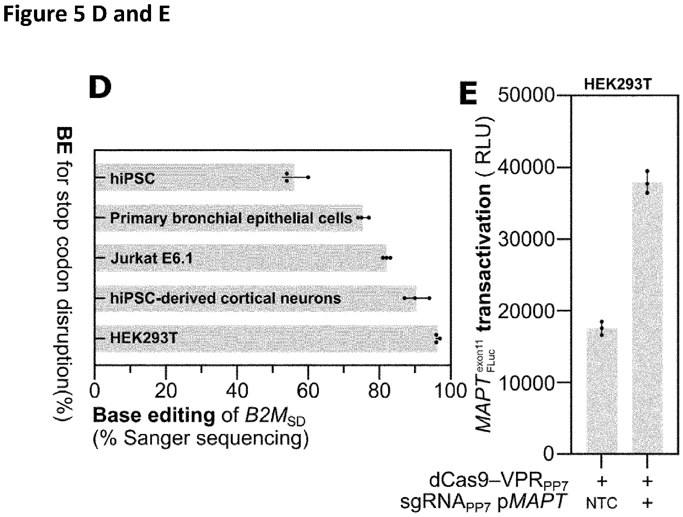

To also utilize the system of this invention for CRISPR activation, the inventors delivered RNPs consisting of dCas9-VPR and a gRNA targeting the *MAPT* transcription start (= *MAPT* promoter) site to a reporter cell line, in which *MAPT* expression can be followed by firefly luciferase (FLuc) signal. The inventors observed a marked increase in bioluminescence signal after 48 hours (Figure 5D).

### Comparison of the delivery system of the present invention to other VLP delivery systems.

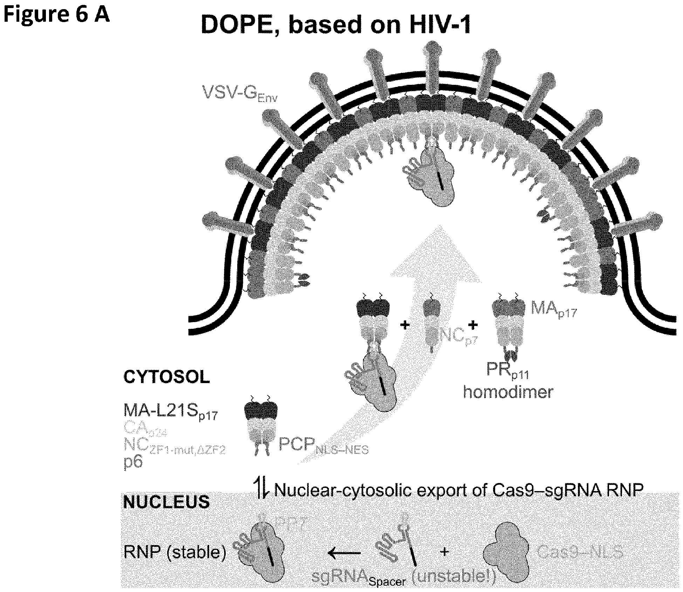

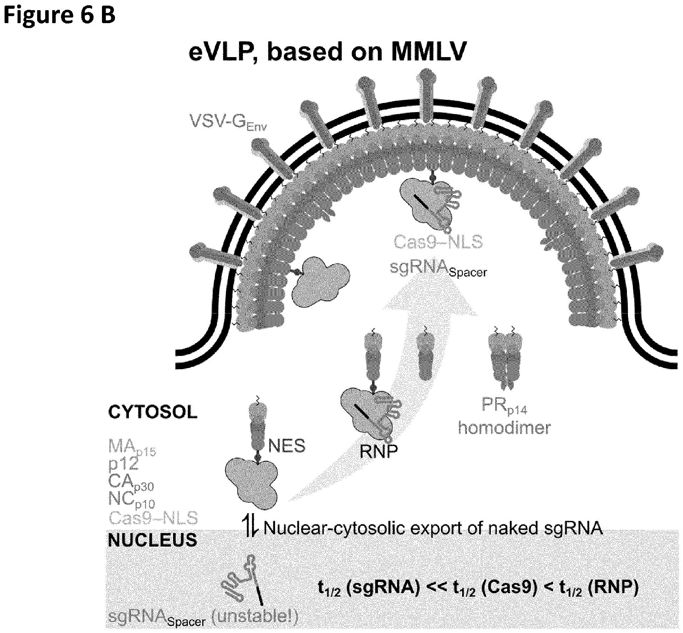

The inventors next sought to compare the present invention to a recently published and widely recognized MMLV-based delivery system from Banskota *et al.* (2022). They introduced "eVLPs", which build on previous iterations of gag-mediated protein delivery (Mangeot *et al.,* 2019; Hamilton *et al.,* 2021) to achieve the transfer of Cas9 or base editors into target cells via direct fusion of a Cas9 effector to an engineered MLV-gag protein. Consequently, the VLP design and cargo loading process is quite different from eVLPs in a couple of critical aspects (see Figures 6A and 6B). As mentioned before, in the present invention the RNP complex is recruited non-covalently via an aptamer handle on the (pe)gRNA (Figure 6A). In contrast, with eVLPs the RNP cargo is covalently linked to Gag, and the release in the target cell is facilitated via additional cleavage sites for the MMI,V protease (Figure 6B). The inventors believe that this key differentiator provides an advantage in terms of efficacy but also for modularity and convenience:
(1) (pe)gRNA stability likely benefits from assembly into RNPs in the nucleus already, that is prior to re-localization to the cytosol via a shuttling component.
(2) having the "loading handle" located on the (pe)gRNA circumvents packaging of "empty" Cas9 effector proteins while the packaging of (pe)gRNAs in complex with an RNP is favored naturally due to the relative short half-life of unbound gRNAs (Wang *et al.,* 2019; Ma *et al.,* 2016; Hendel *et al.,* 2015).
(3) Thirdly, cargo release is independent of any proteolytic cleavage, omitting one kinetic bottleneck entirely. Being independent of the protease activity from Gag-pol also offers the chance of developing and further modifying non-mosaic VLPs. In terms of convenience and modularity, the utilization of Gag-fusion constructs requires modifications to the Gag polyprotein itself, which could affect VLP assembly and may require re-optimization for new cargos.

In contrast, the delivery system of the present invention can be easily repurposed for any (pe)gRNA-guided strategy without changes to the cargo or packaging constructs beyond a designated (pe)gRNA scaffold containing the PP7 aptamer (as demonstrated in Figure 6A).

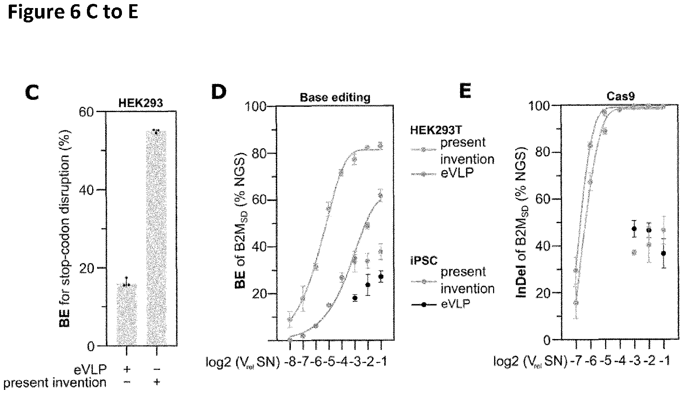

The inventors initially compared the present invention to eVLPs regarding the delivery of ABEs to HEK293 cells carrying the eTLR reporter, which reported an approximate 3-fold increase in editing for the present invention over the BE-eVLP system (Figure 6C). For a more detailed comparison, the inventors next set up titration experiments to compare both systems for ABE8e and Cas9 delivery to target the B2Mlocus in HEK293T and hiPSC, quantifying base edits/InDels via NGS (Figures 6D and 6E). While in HEK293T InDel generation saturates at very low VLP amounts for both systems with comparable performances (Figure 6E), the performance of the present invention in base editing of *B2M* is superior over eVLPs by achieving similar editing efficacy with roughly a magnitude lower volume (Figure 6D). eVLP base editing does not reach saturation within the tested range, it is therefore possible that it matches the efficacy of the present invention if much higher VLP doses are used. Surprisingly, iPSC editing remains at a moderate level regardless of the delivery system or edit type, only marginally (ABE) or not at all responding (active Cas9) to changes in the applied VLP volume (Figures 6D and 6E).

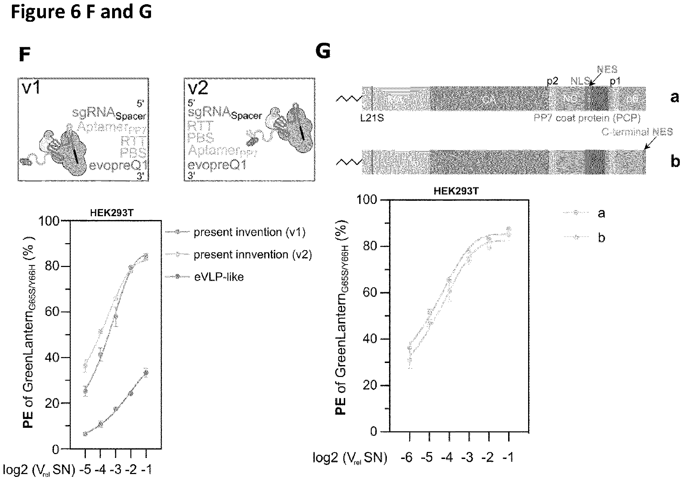

The inventors also attempted a comparison between both systems for PE delivery, even though Banskota *et al.* (2022) did not yet report the delivery of prime editors. As there was no eVLP available for prime editing as of yet, the inventors used the eVLP plasmids provided via Addgene to construct an eVLP-like derivate for PE by exchanging the Cas9 effector domain for the iPE domain within the Gag-Cas9 fusion protein. When tested on the PE reporter from Figure 3A, the inventors observed that it could also mediate prime edits, though not to the extent of the present invention (Figure 6F).

Owing to the complicated cargo release mechanism in eVLPs, the inventors wondered if non-covalent cargo release of the present invention, could still be improved further. The inventors reasoned that if cargo release still poses a bottleneck, the transport of RNPs to the nucleus as a PCP-RNP complex would be equally viable. Assuming that in the chimeric assembly setup the gag-PCP polyprotein is also proteolytically processed just like in eVLPs, a design where the NES is repositioned further downstream to the C-terminus (so that the PCP domain only contains NLS motifs after processing) would utilize both proteolytic cleavage and non-covalent recruitment for cargo release. However, this design did not improve editing efficacy, indicating that cargo release from the non-covalent interaction between PCP and the PP7 aptamer is already sufficient in the recipient cell and does not require an additional proteolytic release (Figure 6G). Nevertheless, other ABP/aptamer pairs with much higher affinity, efficiency of cargo release might benefit from the protease-mediated release.

Due to the exposed nature of the 3'-extension of a pegRNA, comprising the reverse transcribed template (RTT) and the primer binding site (PBS), Nelson *et al.* developed multiple 3' protection motifs using nature-derived RNA pseudoknots (*e.g*., (t)evopreQ1 and tmpknot, Nelson *et al.,* 2022). Albeit the 3'-stability of pegRNAs is extended by such motifs, the inventors think that during its voyage across the producer cell's nucleus and cytosol to the budding location of the VLPs at the plasma membrane, and *vice versa* back in the recipient cell from the endosomal membrane across to cytosol to its destination site in the nucleus would benefit from design considerations that only package RNPs with intact 3'-end, *i.e.,* full-length RTT and PBS. In the current pegRNA design, RNPs with non-functional 3'-truncated pegRNAs could still be packaged and delivered. To prevent this, the inventors investigated moving the PP7 aptamer from the tetraloop position within the gRNA scaffold to the 3' end of the pegRNA downstream of the PBS but upstream of the (t)evopreQ1 motif, effectively restricting packaging of only pegRNAs with an intact 3' RTT and PBS region. Indeed, this resulted in a further increase in editing efficacy (Figure 6F), which now also constitutes the final design for PE delivery of this invention. The inventors did not translate this design to gRNA-based strategies, *i.e.,* nuclease- and base-editing-based genome editing techniques, which natively do not have 3'-extensions. 3'-extensions are usually accompanied by a major decrease in Cas9 activity, while tetraloop modifications, as well as insertions into the first or second stem-loop of the tracrRNA (2^{nd} or 3^{rd} stem-loop of the gRNA, when counting the tetraloop as the 1^{st} stem-loop and the nexus not as stem-loop), are generally well tolerated with only a minor decrease in activity for some targets. Since pegRNAs already have 3'-extensions, insertion of an additional aptamer is also not expected to be detrimental to the activity of prime editing RNPs. Consequently, stem-loop modifications, especially the tetraloop and the stem-loops of the tracrRNA, are better suited for gRNAs to prevent unnecessary loss in Cas9 activity.

So far, the inventors inserted the nuclear export and import sequences (NLS-NES) within the NC domain together with the aptamer-binding protein (ABP). As a consequence, maturation of the VLPs via the maturase/protease domain leads to the release of a modified NC domain comprising the ABP and the NLS-NES, where the NES might lead to a less efficient import of the genome editing RNP complex if the ABP-NLS-NES containing NC domain is still tethered via the ABP-aptamer pair, *e.g*., PCP-PP7 interaction, to the RNP. Thus, the inventors moved the NES portion towards the C-terminus after the budding domain (p6), while the NLS and the ABP were still grafted into the NC domain. When comparing the NC-grafted original and the C-terminal NES version of the VLP system of this invention, no additional benefits could be observed (Figure 6G), indicating that the non-covalent interaction of PCP-PP7 that is sufficient to export the genome editor from the nucleus and package it into VLPs due to the high local concentrations of cargo and packaging components, while in the recipient cells.

### Further engineering of a homomeric VLP assembly setup.

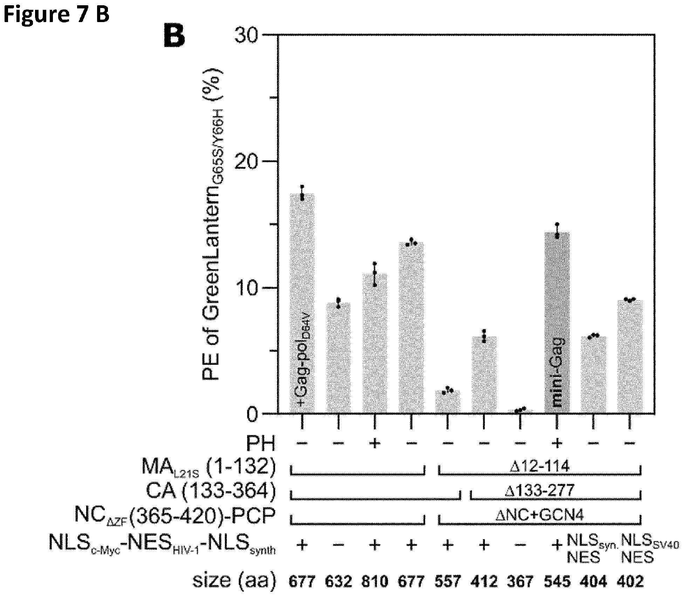

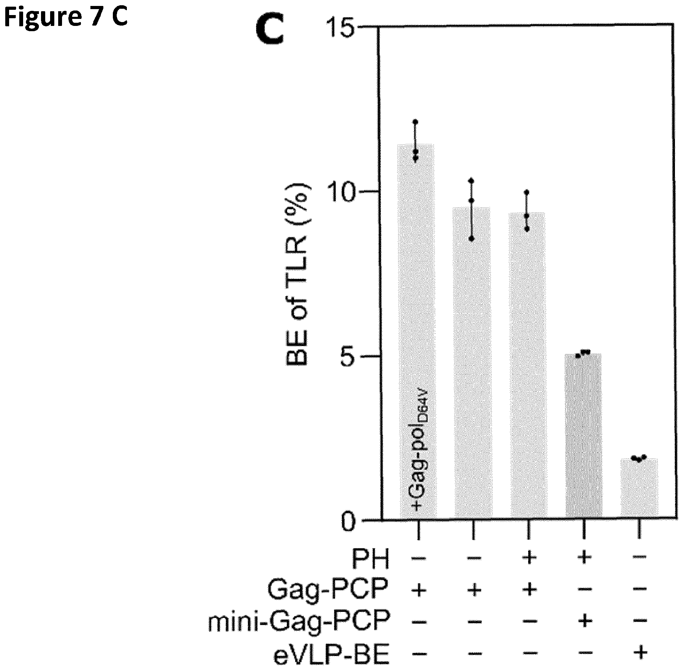

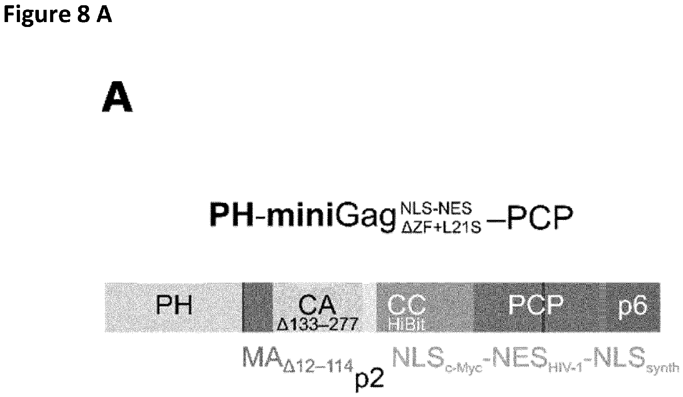

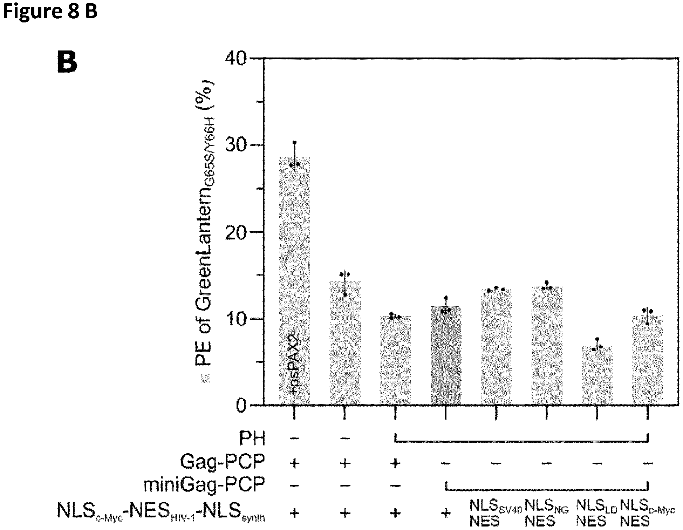

As described in the previous section, the non-covalent cargo recruitment makes the present invention less dependent on HIV-1 protease activity. This provides the opportunity to omit Gag-pol_{D64V} from VLP production entirely, and the inventors concordantly observed that the omission of Gag-pol_{D64V} leads to a substantial yet tolerable decrease in efficacy of RNP delivery. Importantly, a non-chimeric VLP setup allows for further engineering of the gag polyprotein, which the inventors pursued by implementing minimal Gag variants (Accola *et al.,* 2000) with deletions in MA, CA and NC. The inventors tested the PCP fusion of "midi-Gag" (ΔMA12-114, ΔNC) and mini-Gag (ΔMA12-114, ΔCA133-277, ΔNC) (Figure 7A) and observed that while midi-Gag-PCP did not lead to satisfying editing efficacy in the targeted cells, miniGag-PCP seems to be a promising option (Figure 1D, Figure 7B). In addition, the inventors tested an alternative mode of membrane association and fused the phospholipase C-δ1 pleckstrin homology domain (PH) to the N-terminus of various gag variants (Figures 7 and 8).

The inventors observed increased editing, especially with the PH-miniGag variant (Figure 7B). The inventors also hypothesized that the localization equilibrium of the shuttling minimal Gag might have shifted based on its smaller size easing up nuclear import. The inventors, therefore, tested weakened NLS variants and combinations within the NLS-NES-NLS shuttling motif, but none of them led to a significant increase in editing over the initial mini-Gag version.

### Dual-function domains for ultra-high-affinity cargo loading and stabilization.

Previous studies showed that nucleic acid binding domains can be either fused to the C-terminus of Gag, inserted into the NC domain or replacing portions of the NC domain, therefore the inventors set out to find the best possible cargo loading capability with minor impact on Gag's budding capability Indeed, when the inventors compared their choice to replace ZF2 in the NC domain to other possible variants (PCP vs. tandem copies of PCP (tdPCP) and NC ZF2 grafting vs. C-terminal fusion), the inventors' initial design was by far the best variant (PCP_{ZF2} > tdPCP_{C-terminus} > tdPCP_{ZF2} > PCP_{C-terminus}) for the delivery of PE VLPs as mosaic chimera VLPs mixture WT Gag/Gag-pol via psPAX2_{D64V} (Figure 10C). The choice to replace ZF2 in NC was based on the prior knowledge that PCP forms a homodimer and its C-terminal fusion and dimerization might interfere with the budding process by sterically blocking accessibility of the ESCRT-I (via TSG101) and ESCRT-III (via ALIX) to the p6 domain located at the C-terminus of Gag. On the other hand, tdPCP is quite bulky and probably hinders Gag-Gag homo-multimerization due to its spatial requirements, while its tandem nature (tdPCP does not form dimers with other tdPCPs) makes it a more suitable choice for a C-terminal fusion in contrast to the non-tandem counterpart.

In order to improve the editing efficacy per VLP, RNP loading efficiency or half-life have to be improved even further, either by a much higher affinity ABP-aptamer handle or by increasing the effective steady-state concentration of functional non-degraded RNPs. Although protected by a 3' (t)evopreQ1 motif, the 3'-end is probably still the weakest spot of a PE RNP complex. The inventors made use of a CRISPR protein Csy4 (alias Cas6f) from *Pseudomonas aeruginosa* that recognizes a highly conserved 16 nt RNA motif (Csy4 motif C4), wherein the nucleotides 2-16 form a hairpin with a 5 bp stem and a 5 nt loop (SEQ ID NO: 215, 216). When embedded in a stretch of RNA, C4 is bound by Csy4 with an exceptional affinity of 50 pM, which is 20 times higher than the PP7-PCP (K_{d} = 1 nM) interaction. More importantly, Csy4 also precisely cleaves 3'-proximal of the C4 hairpin without leaving any unpaired nucleotides as 3'-overhang. After processing the hairpin, Csy4 will remain bound to the hairpin with a non-altered affinity. The inventors hypothesized that these characteristics make Csy4 an ideal tool to protect the 3'-end of pegRNAs and/or to replace the PCP-PP7 interaction for loading RNPs into the VLPs, or optimally both. When Csy4 is co-expressed in the VLP production cells and the 3'-end of the pegRNA comprises a C4 motif downstream of the PP7, RTT, and PBS but either upstream, downstream of even without the (t)evopreQ1 (Q1) pseudoknot motif, editing efficacy in the recipient cells was dramatically improved compared to controls where C4 is omitted but Csy4 is co-expressed, or *vice versa,* or to the reference VLPs. For all tested conditions, packaging of the pegRNAs into the VLPs was based on the PP7 aptamer inserted either in the scaffold or at the 3' end upstream of Q1 and/or C4 (Figure 10A, Figure 10B). Here, the effect of the improved efficacy and efficiency of the PE VLPs can be solely traced back to the 3'-protection effect of Csy4 first cleaving off the vulnerable ssRNA/polyU stretch (from Pol III termination) and shielding the C4 hairpin 3'-end by clamping it with 50 pM affinity.

To combine the additional layer of 3'-protection with potentially better cargo loading due to the exceptionally high Csy4/C4 affinity, the inventors replaced the grafted PCP domain in the ZF2 region of the NC domain with Csy4 (Figure 1C). The inventors also created an alternative version, where Csy4 is fused after p6 instead, since in contrast to PCP, Csy4 does not dimerize, and thus, interference with the budding process is less likely (Figure 1C). When again applied on the inventors' `blue mGL' reporter system, it outperformed the reference approach using PCP-PP7, independent if Csy4 is either grafted into the NC domain or fused to the C-terminus of Gag (3'-Q1-C4 > 3'-C4-Q1 > 3'-C4, Figure 10C). Of note, these results are somewhat surprising since direct transfection of Csy4 into the reporter cell lines together with iPE and the respective C4-modified pegRNA did not yield any improvement in editing performance indicating Csy4's unique property only beneficial for scenarios where gRNA stability and availability is the major bottleneck. (Figure 11A, Figure 11B).

### Combining VLPs + IDL Vs for HDR-mediated genome editing.

Besides small nucleotides changes which can be mediated by base- and prime-editors, delivery of an HDR-donor template in combination with targeted Cas9 double strand break can be used to introduce large edits or gene insertions.

To enable HDR, Cas9 nuclease and an aptamer-tagged gRNA was delivered as RNPs to HEK293T via the VLP system of the present invention targeting the `blue mGL' reporter system (Figure 12, left). Additionally, an integrase-defective lentivirus encoding the portions of the mGreenLantern chromophore flanked by homology arms to provide a DNA template (after reverse transcription of the delivered RNA transfer vector) for HDR-based repair (Figure 12, right). The VLP as well as the IDLV were pseudotyped with the ecotropic envelope protein of the Moloney murine leukemia virus (MMLV-Env Δ607-622) targeting the murine Slc7a1 receptor (alias mCAT1) that is expressed on the inventors' reporter cell line. To enhance HDR, cells were treated with 0.5 µM AZD7648 during transduction to suppress NHEJ, a repair pathway otherwise competing with HDR.

### Short summary.

Virus-like particles (VLPs) have emerged as promising delivery systems for genome editing *in vivo* due to modular tropism and their intrinsic ability to deliver different cargo types, such as ribonucleoproteins (RNPs), without necessarily introducing a transgene. Herein, the inventors present a novel VLP design derived from the HIV-1 Gag polyprotein, which can efficiently deliver different RNPs to target cells. The inventors demonstrate that the Gag polyprotein (Gag-PCP) specifically recruits (pe)gRNA-loaded RNPs to be packaged as cargo via the interaction between PCP and the PP7 aptamer, which was grafted into to the (pe)gRNA scaffold (but can also be added to the 3' end of pegRNAs specifically). Furthermore, the inventors show that exporting the (pe)gRNA from the nucleus of the producer cell is a critical bottleneck that can be alleviated via shuttling of the Gag protein, specifically by introducing a nuclear localization (NLS) and nuclear export sequence (NES) into the Gag-PCP fusion protein. The delivery system was optimized using the inventors' HEK293T reporter cell lines initially and then successfully applied for the delivery of functional RNPs to primary cell lines, human induced pluripotent stem cells (hiPSCs), and "hard-to-transfect" cell lines such as hiPSC-derived cortical neurons. Simultaneously, the inventors comprehensively demonstrate the modularity and versatility of the inventors' system by delivering various Cas9 effector RNPs to mediate double-strand breaks (DSB), base editing (BE), prime editing (PE) or transactivation.

The cargo recruitment mechanism of the present invention differs quite significantly in comparison to recent Gag-cargo fusion approaches, providing advantages for efficacy and modularity:
(1) The RNP complex is bound and packaged via the PP7 aptamer within the (pe)gRNA, enabling straightforward packaging of virtually any Cas9 effector protein without modification in any way.
(2) Binding to the pre-assembled RNP complex with a nuclear-cytosolic shuttling Gag presumably increases the stability of (pe)gRNAs in the nucleus and prevents packaging of empty Cas9 effector proteins at the same time.
(3) Additionally, the non-covalent cargo recruitment facilitates release in the target cell without further processing, thereby granting independence from any protease activity. Omitting other apparently non-essential enzymatic components of Gag-pol, enables the formation of homomeric, Gag-pol-free VLPs and importantly also allows for the implementation and engineering of structurally altered VLPs such as a minimal-Gag variant.
(4) The present invention includes an additional stabilization of the gRNA, especially pegRNAs, by either co-expressing Csy4/Cas6f together with tagging the (pe)gRNA with a C4 motif inserted into a suitable position close to the 3'-end. Alternatively, Csy4/Cas6f can be grafted into the budding module to fulfill two functions: ABP/aptamer pair for loading of the RNPs and protection of the RNPs at the same time.

In summary, the inventors' novel VLP system of the present invention offers a more efficient, effective, and modular method for the delivery of genome editing complexes as ribonucleoproteins. The optimized design, which utilizes an aptamer-binding protein and dual localization signals, ensures the successful packaging of active RNPs, providing a significant advantage over existing systems.

The option to omit gag-pol from VLP assemblies poses a key innovation compared to other delivery VLP technologies.

The following examples and drawings illustrate the present invention without, however, limiting the same thereto.

### BRIEF DESCRIPTION OF THE DRAWINGS

**Figure 1** *Design of a nucleocytosolic shuttling RNP delivery vehicle.*
   (A) Graphical depiction of the Gag/Gag-pol polypeptide, a lentiviral packaging vector, which codes for Gag (matrix (MA), capsid (CA), nucleocapsid (NC) and p6 domain). The alternative frame codes for the enzymatic components including the functional domains of claim 1, which are highlighted with (a)-(k). (a) plasma membrane-interacting domain, (b) oligomerization domain, (c) membrane-bending domain, (d) shuttling domain, (e) nucleic acid-binding (NAB) domain, (f) budding domain, (g) reverse transcriptase domain, (h) maturation factor, (i) reporter-signal generating domain, (j) nucleic acid-stabilizing domain
   (B) Schematic illustration of the HIV-1 derived shuttling Gag-PCP, whereas PCP serves as an example of a nucleic acid binding (NAB) domain. The first zinc finger motif was inactivated by C>S mutation, while the second zinc finger was replaced by the PP7 coat protein (PCP). The additional nuclear localization and nuclear export sequences mediate Gag shuttling between nucleus and cytosol for efficient packaging of the nuclear localized (pe)gRNA. For (co)-expression of Gag/Gag-Pol_{D64V}, a mutant Gag-Pol_{D64V} was used to inactivate the integrase function (encoded on the Pol frame) and to preclude random integration.
   (C) Schematic illustration of the shuttling Gag- Csy4 fusion protein, where the Csy4 domain was inserted in the NC domain of HIV-1 Gag (top), or fused C-terminally (bottom).
   (D) Illustration of the shuttling-mini-Gag-PCP fusion protein (top) and with the additional N-terminal Pleckstrin (PH) domain (bottom).
**Figure 2** *Delivery of PP7-tagged mRNA.*
   (A) Illustration of mRNA packaging using non-shuttling Gag-PCP. The PP7 aptamer is encoded in the 3'UTR of the cargo mRNA. The VLPs were pseudotyped with VSV-G.
   (B) Effect of the protease inhibitor Darunavir on mGreenLantern mRNA delivery. Darunavir was added during production (prod.) or during transduction (TD) or both (prod. + TD).
   (C) Impact of the inclusion of the L21S mutation in the MA domain of Gag when introduced in Gag-PCP or Gag-pol.
   (D) Comparison of mGreenLantern mRNA delivery via the Gag-pol-PCP or the mosaic set-up using Gag-pol and Gag-PCP.
   (E) Specificity of Gag-PCP towards PP7-tagged aptamers and the effect of microRNA (miR) transfection (depicted by TF) targeting the 3' UTR of mGreenLantern.
   (F) Comparison of mRNA delivery efficiency based on the EPN-24-PCP nanocage or Gag-PCP.

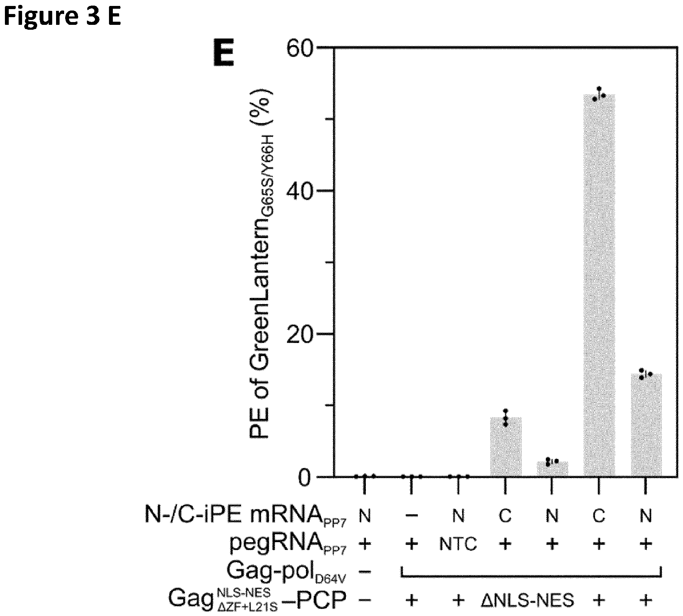

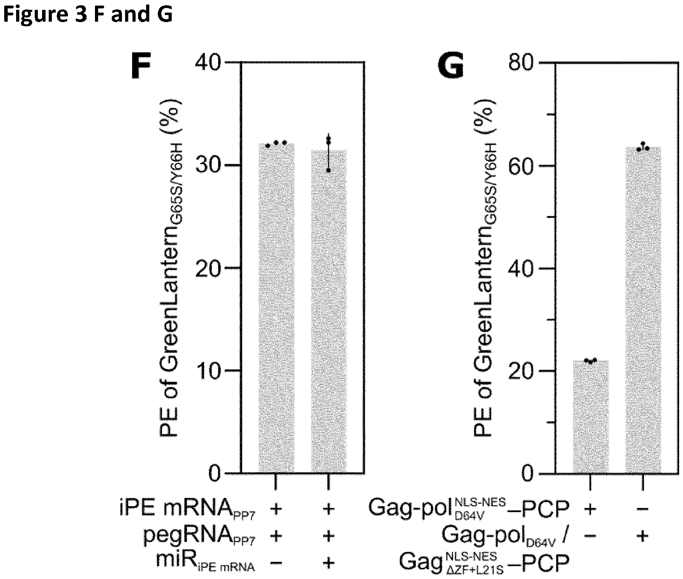

**Figure 3** *Proof of concept of the nucleocytosolic shuttling RNP delivery vehicle, using fluorescence reporter cell lines to quantify prime editing (PE) events.*
   (A) Depiction of packaging and cargo components of the delivery system of the present invention. The cargo RNP, consisting of a Cas9 effector of choice and a corresponding (pe)gRNA. Recruitment of the RNP is mediated via a PP7 aptamer grafted into the (pe)gRNA scaffold, which strongly interacts with the PCP domain in Gag. The cargo RNP complex can already form in the nucleus, stabilizing both components, efficient cargo packaging requires ribonucleoprotein (RNP) translocation from the nucleus into the cytosol, which is enabled by the NLS-NES motif in Gag. After cargo packaging and budding, proteolytic VLP maturation occurs. Notably, cargo release is independent of this process as the RNP is not covalently linked to Gag.
   (B) Prime editing was monitored in a reporter cell line with stably integrated mGreenLantern harbouring two mutations in the chromophore (G65S, Y66H), which shift its excitation range from green to blue. Successful PE reverses the mutations back to wild type, resulting in green fluorescence.
   (C) To determine the limiting component of the prime editor complex, either the mRNA encoding the prime editor protein or the pegRNA were transfected by standard lipofection (TF) while the complementary component was transduced via VLP.
   (D) The different PE efficiencies were obtained for separate packaging of the PP7-tagged pegRNA and iPE mRNA (VLP1 and VLP2) as well as co-packaging of both components into one VLP (VLP1 only). (E) The importance of each component of the delivery system was assessed for PE delivery using the PE reporter. The control conditions demonstrate the importance of the shuttling motif and the PP7:PCP interaction for delivery efficiency.
   (E) Prime editing efficiencies of the nucleocytosolic shuttling Gag-PCP. Quantification of prime editing events in the `blue' mGreenLantern reporter cell line (HEK293T) after delivery of iPE mRNA and PP7-aptamer tagged pegRNAs . N/C indicates the position of the reverse transcriptase (RT) within the iPE complex.
   (F) To determine if the prime editor is delivered as mRNA or rather as RNP, the PE reporter cell line was transfected with a miR targeting the prime editor mRNA. Both, pegRNA and mRNA, were tagged with a PP7 aptamer.
   (G) PE delivery efficiency of the Gag-pol and Gag-PCP mosaic set-up in comparison to a direct insertion of PCP into Gag-pol_{D64V}.
**Figure 4** *Optimization and characterization of the delivery system.*
   (A) Analysis of the impact on PE delivery when the L21S mutation as well as the PCP domain is included in one or both packaging components.
   (B) Inactivation of each component encoded in the 2nd generation packaging vector psPAX2 and the impact of the protease inhibitor Darunavir.
   (C) Optimization of the amount of VSV-G glycoprotein for pseudotyping.
   (D) Optimization of the stoichiometry of Gag-Pol and Gag-PCP (packaging plasmids) as well as the ratio of packaging plasmids to cargo plasmids.
   (E) Titration of the cargo plasmids encoding the pegRNA and nCas9:RT.
**Figure 5** *Delivery of alternative RNP effectors for introduction of InDels, base editing (BE) or transactivation of an endogenous target locus.*
   (A) The enhanced traffic light reporter (eTLR) was used to quantify DSB events. By targeting the stop codon region, all potential InDel events lead to a read-through activating eUnaG in any of the three downstream ORFs. The reporter can also be used to detect a successful base conversion within that same stop codon, leading to continued translation of the downstream eUnaG reporter.
   (B) Delivery of Cas9 and PP7-tagged gRNA as RNPs. Quantification of InDels in HEK293 reporter cell line via flow cytometry.
   (C) Delivery of *ABE8e_{NLS}* and PP7-tagged gRNA as RNPs. Quantification of base conversion within the TLR reporter in cortical neurons via flow cytometry.
   (D) Quantification of base conversion upon delivery of an ABE8e_{NLS} RNP into various cell lines. The gRNA targets the splice donor (SD) of the E2Mlocus leading to a functional KO of the *B2M* locus.
   (E) Transactivation of the *MAPT* gene expression by delivery of a dCas9-miniVPR_{NLS} RNP into a HEK293T cell line the inventors described previously (Truong *et al., 2021). MAPT* expression of the reporter cell line can be followed by luciferase expression.
**Figure 6** *Comparison of the delivery system of the present invention to e VLPs.*
   (A) Packaging modalities: Cas9 binds and stabilizes the (pe)gRNA in the nucleus; nuclear cytosolic shuttling of Gag-PCP enables binding of the fully assembled RNP via PCP:PP7 interaction. The system allows for modular packaging of essential any RNP complex if the (pe)gRNA is tagged with the PP7 aptamer.
   (B) eVLP consists of a chimeric fusion of Gag and a genome editing effector protein of choice. The fusion protein shuttles between nucleus and cytosol to package the unprotected (pe)gRNA. In contrast to the present invention, packaging of the Cas protein without (pe)gRNA is possible, the genome editor is released after budding upon proteolytic cleavage of the protease encoded in Gag-pol.
   (C) Comparison of base editing efficacy regarding editing the eTLR reporter.
   (D) Titration curves of base conversion of the *B2M* splice donor after delivery of the present invention and eVLPs to HEK293T or iPSC. Quantification of base editing via NGS.
   (E) Quantification of InDels in the B*2M* locus after Cas9-RNP delivery into HEK293T and iPSC.
   (F) Comparison of RNPs for prime editing with a derivate of the delivery system of the present invention and eVLP system from the Liu group (eVLP-like), where the inventors exchanged the Cas9 effector domain for the iPE domain within the Gag-Cas9 fusion protein. The eVLP-like iPE was co-delivered with a pegRNA to correct the mutations of the `blue' mGreenLantern reporter. v1 refers to the co-delivery of iPE and the pegRNA from previous experiments, with the PP7 aptamer grafted into the scaffold. In v2, the inventors translocated the PP7 aptamer to the 3' of the pegRNA preventing packaging of 3' truncated pegRNAs.
   (G) Illustration of repositioning the NES motif from the shuttling domain to the C-terminus (top). Comparison of both NES positions regarding delivery of prime editors to the `blue' mGreenLantern reporter cell lines (bottom).
**Figure 7** *Engineering of a minimal Gag variant for delivery of functional RNPs.*
   (A) Schematic of WT HIV-1 Gag (top) and minimal Gag (bottom) which is truncated in MA and CA. The NC domain is completely replaced by the shuttling motif and PCP. To improve particle formation at the membrane, a Pleckstrin homology domain (PH) was fused to the N-terminus of Gag.
   (B) Set up of a miniGag variant (darker green) with deletions in MA, CA and NC in combination with the phospholipase C-δ1 pleckstrin homology domains (PH) for membrane association and a GCN4 coiled coil for oligomerization, as well as alternative nuclear-cytosolic shuttling motifs. Quantification of the prime editor complex delivery to reverse the G65S/Y66H mutation of the mGreenLantern reporter via flow cytometry.
   (C) Comparison of shuttling PH-miniGag against Gag-PCP- and eVLP- delivery regarding base editing efficiency of the TLR reporter locus in HEK293.
**Figure 8** *Optimization of the shuttling motif for minimal Gag variants.*
   (A) Schematic depiction of shuttling PH-miniGag-PCP carrying multiple deletions (ΔMA12-114, ΔCA133-277, ΔNC).
   (B) Combination of several NLS-NES shuttling motifs with miniGag-PCP.
**Figure 9** *Workflow of VLP production process.*
   (A) Co-transfection of HEK293T with packaging plasmid, cargo plasmid and pseudotyping plasmid. The cargo plasmid encodes for the PP7 aptamer-tagged (pe)gRNA and a suitable genome editing effector protein. 48-72 h post-transfection of the producer cells, the supernatant (SN) containing the VLPs, is collected and sterile-filtered to remove cell debris and left-over transfection reagents. Subsequently, the filtered SN is concentrated by using a molecular weight cutoff filter (100 kDa).
   (B) For pseudo-transduction of the target cells, the supernatant is applied to the cells without exceeding 20% of the total volume of the receiver cell's original growth medium.
   (C) For optimizing the VLP system, cells were analyzed 72 h post-pseudo-transduction via targeted amplicon sequencing of the respective targets or FACS analysis if fluorescence reporter cell lines were used.
**Figure 10** *Implementation of the C4 aptamer and Csy4 enzyme to optimize the delivery system.*
   (A) Depiction of packaging and cargo components of the delivery system additionally utilizing the Csy4 ABP-aptamer system either only as protection for the pegRNA 3' end (left) or for both protection and recruitment of the pegRNA simultaneously (right), replacing PCP-PP7. To increase pegRNA stability, a Csy4-aptamer (C4) was introduced at different positions of PP7-aptamer modified pegRNAs in combination with tEvopreQ1 (Q1) and co-expressed with the Csy4/Cas6f protein in the VLP producer cell line. Prime editing efficacy was assessed using the blue-shifted mGreenLantern reporter system and quantifying the percentage of green cells and median fluorescence intensity separately or combined.
   (B) The omission of Csy4 protein expression in the VLP producer cell line was assessed using the best-performing C4 aptamer pegRNA variant from (A)Prime editing efficacy was assessed using the blue-shifted mGreenLantern reporter system and quantifying the percentage of green cells and median fluorescence intensity separately (left) or combined (right).
   (C) To switch from PP7-mediated recruitment to Csy4-mediated recruitment, PCP was exchanged for the Csy4 protein, which was grafted into the NC domain (as PCP before) or placed at the C-terminus of the Gag-PCP fusion protein. For the pegRNA, the PP7 aptamer was correspondingly omitted and the C4 aptamer was placed at different positions in the 3' end. Prime editing efficacy was referenced against multiple PCP-PP7 configurations, again using the blue-shifted mGreenLantern reporter system and quantifying the percentage of green cells and median fluorescence intensity separately (left) or combined (right).
**Figure 11** *Transfection of HEK293T to test pegRNAs comprising the C4 aptamer as stabilization motifs for prime editing.*
   (A) To assess the effect of the C4 aptamer at the 3' of pegRNAs in transient transfection settings, prime editing efficiencies were obtained in absence and presence of the Csy4 protein.
   (B) Quantification of prime editing efficiencies for pegRNAs containing the PP7 and C4 aptamer with varying position at the 3'-end. The expression of Csy4 and iPE is coupled via a ribosomal skipping mechanism (2A).
**Figure 12** *Delivery of a DNA donor template for homology-directed repair of the 'blue* ' *mGreenLantern reporter.*

The `blue' mGreenLantern (G65S, Y66H) reporter cell line shows blue fluorescence unless the chromophore mutations are reversed, e.g., via prime editing or after a targeted double-strand break at the chromophore site followed by homologous recombination (HR or HDR) mediated repair using a donor DNA template coding for the reversed chromophore mutations or delivery (left).

To enable HDR, Cas9 and a PP7-tagged gRNA targeting the reporter locus was delivered to HEK293T via Gag-PCP VLPs. Additionally, a psi-tagged RNA encoding the mGreenLantern chromophore flanked by homology arms and long terminal repeats (LTRs) was packaged and reverse transcribed via an integrase-deficient (D64V) lentivirus (IDLV) to provide a DNA template for HDR (right). The VLP as well as the IDLV were pseudotyped with the ecotropic envelope protein of the murine leukemia virus (MLV-Env Δ607-622) targeting the murine *Slc7a1* (mCAT1) receptor. To enhance HDR, cells were treated with 0.5 µM AZD7648 prior to transduction.

### EXAMPLES

### EXAMPLE 1 Materials and Methods

### 1. Molecular Cloning and DNA analysis

### 1.1 PCR

Single-stranded primer deoxyribonucleotides (Integrated DNA Technologies (IDT)) were resolubilized 100 µM) in nuclease-free water. PCR reaction with plasmid and genomic DNA templates was performed with Platinum SuperFi II PCR Master Mix (Thermo Fisher Scientific) according to manufacturer's protocol. PCR reactions were purified by DNA agarose gel electrophoresis and subsequent DNA extraction using Monarch^{®} DNA Gel Extraction Kit (New England Biolabs (NEB)).

### 1.2 DNA digestion with restriction endonucleases

Samples were digested with NEB restriction enzymes according to the manufacturer's protocol in a total volume of 40 µl with 1-3 µg of plasmid DNA. Afterwards, DNA fragments were purified by agarose gel-electrophoresis and subsequent purification using Monarch^{®} DNA Gel Extraction Kit (NEB).

### 1.3 Ligation and Gibson assembly

Concentrations of agarose-gel purified DNA fragments were determined by a spectrophotometer (NanoDrop 1000, Thermo Fisher Scientific). Ligations were carried out with 50-100 ng backbone-DNA (DNA fragment containing the ori) in 20 µl volume, with molar 1: 1-3 backbone:insert ratios, using T4 DNA ligase (Quick Ligation^{™} Kit, NEB) at room temperature for 5-10 min. Gibson assemblies were performed with 75 ng backbone DNA in a 15 µl reaction volume and molar 1: 1-5 backbone:insert ratios, using NEBuilder^{®} HiFi DNA Assembly Master Mix (2x) (NEB) for 20-60 min at 50 °C.

### 1.4 DNA agarose gel-electrophoresis

1% (m/m) agarose (Agarose Standard, Carl Roth) gels were prepared in 1x TAE-buffer and 1:10.000 SYBR Safe stain (Thermo Fisher Scientific). Gel electrophoreses were carried out for 20-40 min at 100 V. For size determination, 1 kb Plus DNA Ladder (NEB) was used. DNA samples were mixed prior to loading with Gel Loading Dye (Purple, 6x) (NEB).

### 1.5 Bacterial strains (E. coli) for molecular cloning

Chemically competent Stable cells (NEB^{®} Stable) were used for transformation of circular plasmid DNA. For plasmid amplification, carbenicillin (Carl Roth) was used as a selection agent at a final concentration of 100 µg/ml. All bacterial cells were incubated in Lysogeny Broth (LB) medium or on LB agar plates including the respective antibiotics.

### 1.6 Bacterial transformation with plasmid DNA

Transformation was performed by mixing 1-5 µl of ligation or Gibson reaction with 50 µl thawed, chemically competent cells, which were incubated on ice for 30 min before heat shocking at 42 °C for 30 s. Afterwards, cells were incubated on ice for 5 min and finally mixed with 450 µl SOC-medium (NEB). Transformed cells were then plated on agar plates containing an appropriate type and concentration of antibiotics according to the supplier's information. Plates were incubated overnight at 37 °C.

### 1.7 Plasmid DNA purification and Sanger sequencing

*E. coli* colonies with correct potential constructs were inoculated from agar plates in 2 ml LB medium at 37 °C with the respective antibiotics and incubated for at least 6 h or overnight. Plasmid DNA was extracted with Monarch^{®} Plasmid Miniprep Kit (NEB) according to the manufacturer's protocol and sent out for Sanger sequencing (GENEWIZ, Azenta Life Sciences). Sanger sequencing validated clones were inoculated in 100 ml LB medium overnight at 37 °C containing the respective antibiotic selection agent. Plasmid DNA was extracted using the Plasmid Maxi Kit (QIAGEN).

### 1.8 Genomic DNA isolation

72 hours after transfection or transduction in 96-well format, genomic DNA was isolated with the Quick-DNA 96 Kit (Zymo Research) according to the manufacturer's protocol with an elution volume of 30 µl.

### 1.9 Amplicon PCR and purification

PCR was performed as described above using ~50 ng of gDNA and appropriate primers for each target. Amplicon lengths were designed to approach 250 bp for sequencing. PCR purification was performed using the DNA Clean & Concentrator-5 Kit (Zymo Research) according to the manufacturer's protocol with an elution volume of 30 µl

### 1.10 Amplicon sequencing and analysis

Following an initial PCR on the genomic DNA, a second outer PCR using barcoded primers was performed. PCR products of each experiment were purified as described above, normalized, and pooled. The mixture was gel-purified, normalized to 20 ng/µl, and submitted for Amp-EZ sequencing (GENEWIZ, Azenta Life Sciences). The resulting fastq files containing paired reads were analyzed with Geneious via barcode separation and CRISPR editing analysis within the entire range covered by reads or at least the full sequence area between the genomic primer binding sites.

### 2. Mammalian cell culture

### 2.1 Cell lines and cultivation

Cells were cultured at 37 °C, 5% CO₂, and H₂O saturated atmosphere.

HEK293T (ECACC: 12022001, Sigma-Aldrich) and HEK293 eTLR (originate from HEK293, ECACC 85120602) cells were maintained in advanced DMEM (Gibco^{™}, Thermo Fisher Scientific) supplemented with 10% FBS (Gibco^{™}, Thermo Fisher Scientific), GlutaMAX (Gibco^{™}, Thermo Fisher Scientific), 100 µg/ml Penicillin-Streptomycin (Gibco^{™}, Thermo Fisher Scientific), 10 µg/ml Piperacillin (Sigma-Aldrich) and 10 µg/ml Ciprofloxacin (Sigma-Aldrich). Cells were passaged at 90% confluence by removing the medium, washing with DPBS (Gibco^{™}, Thermo Fisher Scientific) and detaching the cells with Accutase^{®} solution (Gibco^{™}, Thermo Fisher Scientific). Cells were then incubated for 5-10 min at room temperature until a visible detachment of the cells was observed. Accutase^{™} was subsequently inactivated by addition of pre-warmed DMEM including 10% FBS and all supplements. Cells were then transferred into a new flask at an appropriate density or counted and plated on 96-well or 6-well format for plasmid transfection or VLP transduction.

Jurkat E6.1 cells (ECACC 88042803) were maintained in RPMI media 1640 (Gibco^{™}, Thermo Fisher Scientific) with GlutaMAX^{™} (Gibco^{™}, Thermo Fisher Scientific) and

100 µg/ml Penicillin-Streptomycin (Gibco^{™}, Thermo Fisher Scientific). For transduction, they were seeded to 96-well format at 25,000 cells/well. The proper density was achieved by centrifugation 200 rcf for 2 min and subsequent dilution RPMI 1640 to the desired density.

### 2.2 hiPSC cultivation

hiPSCs were invariantly incubated in Essential 8 Flex medium (Gibco, Thermo Fisher Scientific) at 37 °C under 5% CO₂ saturation on (v/v) Vitronectin-coated (A31804, Thermo Fisher Scientific) plates. At 70% confluency, the cells were subcultivated with StemMACS Passaging Solution XF (Miltenyi Biotec) by incubating the cells for 6 min at room temperature. Subsequently, StemMACS Passaging Solution XF was aspirated, and cells were collected by adding 1 ml Essential 8 Flex medium to the cells. Collected cells were transferred to a flask containing 2 ml Essential 8 Flex medium and seeded on 96-, 48-, 24- or 6-well plates at the appropriate cell densities.

### 2.3 hiPSC-derived cortical neurons

hiPSCs were plated on multiwell plates coated with 1% (v/v) Geltrex (A1413302, Gibco, Thermo Fisher Scientific) 24 h before neuronal initiation, maintained in Essential 8 medium (A2858501, Gibco, Thermo Fisher Scientific). For neuronal initiation, smNPC maintenance medium-a 1:1 composition of N2 and B27 medium supplemented with 100 ng µl⁻¹ FGF8b (130-095-740, Miltenyi Biotec), 100 nM LDN193189 (130-103-925, Miltenyi Biotec) and 10 µM SB431542 (S1067, Selleck Chemicals)-was added to the cells. N2 medium was composed of DMEM/F12 + GlutaMax (10565018, Gibco, Thermo Fisher Scientific), 1×N2 supplement (17502048, Gibco, Thermo Fisher Scientific), 5 µg ml⁻¹ insulin (I9278-5ML, Sigma-Aldrich), 100 µM MEM non-essential amino acids (11140035, Gibco, Thermo Fisher Scientific), 50 µM β-mercaptoethanol (M6250, Sigma-Aldrich) and 0.5×(50 U ml⁻¹) penicillin-streptomycin (15140122, Gibco, Thermo Fisher Scientific). B27 medium was composed of neurobasal medium (21103049, Gibco, Thermo Fisher Scientific), 1×B27 supplement (11530536, Gibco, Thermo Fisher Scientific), 1×GlutaMax Supplement (35050038, Life Technologies) and 0.5× (50 U ml⁻¹) penicillin-streptomycin. On day 14, the neuroepithelial sheet was replated onto a six-well plate, coated with 15 µg ml⁻¹ poly-L-ornithine hydrobromide and 10 µg ml⁻¹ laminin. On day 21, medium was aspirated, and cells were washed once with Dulbecco's phosphate-buffered saline (DPBS) (14190169, Gibco, Thermo Fisher Scientific). Cells were detached using Accutase (6964, Sigma-Aldrich) under incubation for 10 min at 37 °C. The Accutase reaction was stopped by transferring the cells into a 15 ml Falcon containing 6 ml DMEM/F-12, GlutaMax. Cells were sedimented by centrifugation at 200 r.c.f. for 5 min, and cell pellets were resuspended in smNPC maintenance medium supplemented with 100 µM Y-27632 dihydrochloride (ALX-270-333-M005, Enzo Life Sciences) and counted using a Neubauer improved, precision disposable plastic hemocytometer C-chip (PK361, Carl Roth). Appropriate cell densities were seeded and cells were cultured in smNPC maintenance medium supplemented with 100 µM Y-27632 dihydrochloride for 24 h at 37 °C under invariant 5% CO₂ saturation. The next day, the medium was aspirated and neuronal differentiation was initiated by adding neuronal induction medium - a 1:1 composition of N2 and B27 medium, supplemented with 200 µM ascorbic acid (10389701, Th. Geyer) and 20 ng ml⁻¹ brain-derived neurotrophic factor (130-093-811, Miltenyi Biotec). In the case of detachment of the neuronal network, cells were reseeded onto new plates coated with 15 µg ml⁻¹ poly-L-omithine hydrobromide (P3655, Sigma-Aldrich) and 10 µg ml⁻¹ laminin (L2020-1MG, Sigma-Aldrich). Neuronal networks were dissociated into single cells using Accutase. Cells were then carefully washed once with DPBS, and incubated with Accutase for 15 min at 37 °C under invariant 5% CO₂ saturation. The Accutase reaction was stopped by transferring the cell solution into a 15 ml Falcon tube, containing 3 ml neuronal induction medium supplemented with 100 µM Y-27632 dihydrochloride. Cells were released from Y-27632 dihydrochloride by a medium change 24 h after reseeding.

### 2.4 Plasmid transfection for VLP production

HEK293T cells were seeded on a 6-well plate in 3 ml advanced DMEM with a density of 200,000 cells/ml. One day post seeding, the cells were transfected with the packaging plasmid (333 ng), pseudotyping plasmid (200 ng) and the cargo plasmids. The first cargo plasmid encodes for the PP7 aptamer-tagged (pe)gRNA (500 ng) as well as a suitable genome editing effector protein (167 ng). The lipofection was carried out according to manufacturer's protocol (jetOPTIMUS, Polyplus). DNA amounts were kept constant in all transient experiments to yield reproducible complex formation and comparable results. In 6-well plates, a total amount of 1.2 µg of plasmid DNA was used per well. After 48-72 h cultivation of the producer cells, the supernatant (SN) was collected.

### 2.5 Isolation and transduction of VLPs

The supernatant (SN), containing the VLPs, was sterile-filtered to remove cell debris and remaining lipofection reagents. Subsequently, the filtered SN was concentrated to approximately 1/30 of the original volume using a molecular weight cutoff filter (100 kDa). For pseudo-transduction (TD), the respective reporter cell line was seeded on a 96-well plate (25,000 cells in 200 µl advanced DMEM). 6h post seeding, the supernatant of one 6-well production was applied to a triplicate of receiver cells on the 96-well plate without exceeding 20% of the total volume of the receiver cell's original growth medium. For optimizing the VLP system, cells were analyzed 72 h post TD when delivering an RNP complex and 24h post TD when delivering mRNA.

Storage capabilities of the VLPs vary depending on pseudotyping. VSV-G pseudotyped VLPs can be stored at 4 °C for at least three days without significant loss of infectivity. For other pseudotyped VLPs the inventors recommend to shock-freeze the VLPs using liquid nitrogen.

### 2.6 Generation of stable cell lines carrying the fluorescent prime editing reporter via CRISPR/Cas9

Cell lines were created by cloning the reporter coding for the blue-shifted mGreenlantern2 mutant (G65S, Y66H) with a CAG-promoter and a bovine growth hormone (bGH) polyadenylation signal (pA) was cloned with homology arms for a safe harbour locus of choice. To create a HEK293T reporter cell line, cells were transfected with the CRISPR donor plasmid as well as a second plasmid encoding Cas9 and the corresponding gRNA. Cells were transfected according to the manufacturer's protocol (jetOPTIMUS, Polyplus) 24 hours post-seeding on a 6-well plate (600k cells in 3 ml per well) in the presence of 0.5 µM AZD7648 (HY-111783; MedChemExpress), a DNA-PCcs inhibitor, and a CAG-promoter-driven i53, a 53BP1 inhibitor, to inhibit NHEJ and shift the DNA repair towards HDR (see also Truong *et al.,* 2022; Truong *et al.,* 2023). The surviving polyclonal population was monoclonalized using limiting dilution in 96-well plates, selected clones were genotyped to determine homozygosity.

### 2.7 Immunoblot analysis

Cells were lysed with the appropriate volume of M-PER (Thermo Fisher Scientific), including protease inhibitors (Halt Protease Inhibitor Cocktail, Thermo Fisher Scientific), according to the manufacturer's protocol. Cleared lysates were then equalized against the relative protein concentration determined using the NanoDrop 1000 (Thermo Fisher Scientific) and diluted with M-PER. Equalized lysates were prepared for SDS-gel-electrophoresis using XT sample buffer (Bio-Rad) and XT reducing agent (Bio-Rad) and denatured at 70 °C for 10 min or 95 °C for 5 min. Samples were loaded in 18-well 4-12% Criterion XT Bis-Tris Protein Gel, and electrophoresis was run at 200 V for 1 h in XT MOPS running buffer (Bio-Rad). Molecular mass markers (Amersham ECL Full-Range Rainbow Molecular Weight Markers, GE Healthcare Life Sciences, General Electric) were used in parallel to confirm the different protein species. Subsequently, an immunoblot was performed onto an Immobilon-P PVDF membrane (Merck) with a wet blotting system (Criterion Blotter, Bio-Rad) in ice-cold Towbin buffer (Bio-Rad) with 20% (v/v) methanol (Carl Roth) at 100 V for 30 minutes in an ice water reservoir or overnight (15 V at 4 °C). Next, the free valences on the PVDF membrane were blocked in blocking buffer containing 5% (m/v) skimmed milk (Carl Roth) in TBS-T (pH 7.6) with 0.1% (v/v) Tween-20 (Sigma-Aldrich) at room temperature for 1 h. Antibodies were diluted at the indicated dilution factors in blocking buffer and either incubated at room temperature for 2 h or overnight at 4 °C, followed by at least three washing steps (room temperature, 5 min, 60 r.p.m.). The HRP-conjugated secondary antibody (Abcam) was also diluted in blocking buffer (1:20,000) and subsequently washed again with TBS-T at least four times. HRP detection was performed using the SuperSignal West Femto Maximum Sensitivity Substrate or SuperSignal West Atto Ultimate Sensitivity Substrate (Thermo Fisher Scientific) on the Fusion FX7/SL advanced imaging system using FusionCapt Advance SL4 v. 16.09b (Vilber Lourmat). The primary and secondary antibodies used were as follows: HIV-1 p55 Gag (full length) antibody (1:2,000, #ABIN2452023, antibodies-online GmbH), AC-15 mouse anti-beta-actin (HRP-coupled, 1:100,000, ab6276, Abcam), goat anti-mouse IgG H&L (HRP-coupled, 1:20,000, ab97023, Abcam), goat anti-rat IgG H&L (HRP-coupled, 1:20,000, ab97057, Abcam) and goat anti-rabbit IgG H&L (HRP-coupled, 1:20,000, ab6721, Abcam). Quantification was performed using Image Lab (v.6.1.0 build 7, Bio-Rad).

### 2.8 FACS analysis.

FACS analysis was performed on the BD FACSaria II system (controlled with the BD FACSDiva Software (v.6.1.3, BD Biosciences)). Briefly, the main population of the cells was gated first according to their FSC-A and SSC-A. Secondly, single cells were gated using FSC-A and FSC-W. The final gate (green fluorescence) was used to determine the proportion of successfully edited/transduced cells.

### 2.9 Bioluminescence quantification

For bioluminescence bulk quantifications, cells were plated (and pseudo-transduced) in 96-well format. For bioluminescence detection of secreted NLuc, the supernatant was sampled (80 µL) at the indicated time points and detected using the Nano-Glo^{®} Luciferase Assay System (Promega) on the Centro LB 960 (Berthold Technologies) plate reader with 0.5 s acquisition time. For dual-luciferase read-out using Nano-Glo^{®} Dual-Luciferase^{®} Reporter Assay System (Promega), NLuc and FLuc signals were read out on-plate 48 hours post-transfection. Signals were obtained with 0.5 s acquisition time 10 min after the addition of reagent 1 (ONE-Glo^{™} EX Luciferase) for FLuc and 10 min after addition of reagent 2 (NanoDLR^{™} Stop & Glo^{®}) for NLuc. Reagent 2 includes an FLuc inhibitor.

### 2.10 Statistics

Statistics were calculated with Prism 9 (Graphpad) as indicated.

### 2.11 Protein domains

| **Original organism (referring to aa sequence)** | **Name** | **Mutation** | **type** | **length** | **SEQ ID NO:** |
|---|---|---|---|---|---|
| Streptococcus pyogenes | Cas9 nickase v1 | H840A | aa | 1367 | 40 |
| | | | nt | 4101 | 118 |
| Streptococcus pyogenes | Cas9 nickase v2 | N863A | aa | 1367 | 41 |
| | | | nt | 4101 | 119 |
| Streptococcus pyogenes | Cas9 nickase v3 | H840A, N863A | aa | 1367 | 42 |
| | | | nt | 4101 | 120 |
| Sulfolobus solfataricus | Sso7d | | aa | 63 | 43 |
| | | | nt | 189 | 121 |
| Sulfolobus solfataricus | Sso7d with inactivated | E35L | aa | 63 | 44 |
| | | | nt | 189 | 122 |
| | RNAse activity | | | | |
| Moloney Murine Leukemia Virus | M-MLV reverse transcriptase with enhancing mutations | H8Y, D200N, T306K, W313F, T330P, L603W | aa | 677 | 45 |
| | | | nt | 2031 | 123 |
| Moloney Murine Leukemia Virus | M-MLV reverse transcriptase with enhancing mutations and without RNAse H domain | H8Y, D200N, T306K, W313F, T330P | aa | 495 | 46 |
| | | | nt | 1485 | 124 |
| Eubacterium rectale | MarathonRT | | aa | 426 | 47 |
| | | | nt | 1278 | 125 |
| Synthetic | superNLS1 | | aa | 22 | 48 |
| | | | nt | 66 | 126 |
| Synthetic | superNLS2 | | aa | 35 | 49 |
| | | | nt | 105 | 127 |
| Synthetic | Synthetic NLS | | aa | 9 | 50 |
| | | | nt | 27 | 128 |
| Homo sapiens | MYC NLS | | aa | 7 | 52 |
| | | | nt | 27 | 130 |
| Synthetic | XTEN linker | | aa | 27 | 55 |
| | | | nt | 81 | 133 |
| PP7 bacteriophage | PP7 coat protein (PCP) non-aggregating mutant | the loop from C67 to E74 replaced by SG | aa | 121 | 56 |
| | | | nt | 363 | 134 |
| PP7 bacteriophage | Tandem copies of PCP non-aggregating mutant | the loop from C67 to E74 replaced by SG for both PCP domains | aa | 247 | 57 |
| | | | nt | 741 | 135 |
| M2 bacteriophage | MS2 coat protein non-aggregating high affinity mutant | N55K for high affnity; A81G and V75E for non-aggregation mutant | aa | 129 | 58 |
| | | | nt | 387 | 136 |
| Synthetic | FLAG tag | | aa | 8 | 59 |
| | | | nt | 24 | 137 |
| HIV-1 | gag | | aa | 500 | 60 |
| | | | nt | 1500 | 138 |
| HIV-1 | gag-L21S | L21S permanently activates myristoyl switch | aa | 500 | 61 |
| | | | nt | 1500 | 139 |
| Pseudomonas aeruginosa | Csy4 | | aa | 187 | 64 |
| | | | nt | 561 | 142 |
| HIV-1 | NES | | aa | 11 | 65 |
| | | | nt | 33 | 143 |
| | CANTDcb1 | | aa | 123 | 66 |
| | | | nt | 369 | 144 |
| | HiBiT | | aa | 11 | 67 |
| | | | nt | 33 | 145 |
| | PLC-PHP | | aa | 132 | 68 |
| | | | nt | 396 | 146 |
| | GCN4 | | aa | 33 | 69 |
| | | | nt | 99 | 147 |
| | trimer forming GCN4 mutant | | aa | 33 | 70 |
| | | | nt | 99 | 148 |

### 2.12 Proteins and protein chimeras

| **Name** | **type** | **SEQ ID NO.** |
|---|---|---|
| Cas9-N863A:HA:superNLS2:XTEN:MLV-RT-x6:SV40-NLS | aa | 28 |
| | nt | 108 |
| Cas9-N863A:HA:superNLS2:XTEN:MLV-RT-x6-ΔRNAseH | aa | 29 |
| | nt | 109 |
| MLV-RT-x6:SV40-NLS:XTEN:Cas9-H840A:superNLS2:FLAG | aa | 30 |
| | nt | 110 |
| MLV-RT-x6-ΔRNAseH:SV40-NLS:XTEN:Cas9-H840A:superNLS2:FLAG | aa | 31 |
| | nt | 111 |
| MLV-RT-x6:SV40-NLS:XTEN:Cas9-H840A:superNLS2:FLAG:Sso7d-E35L | aa | 32 |
| | nt | 112 |
| BPNLS-ABE8e-SpCas9-D10A-superNLS2 | aa | 33 |
| | nt | 113 |
| Cas9-N863A-HA-2xSV40NLS | aa | 35 |
| | nt | 115 |
| eTLR coding sequence (contains frameshift sites! All three frames play a role) | aa | 36, 37, 38 |
| | nt | 116 |
| gag-L21S(ZF1(C>S)-ΔZF2):HiBiT:NLS-NES-NLS:PCP | aa | 1 |
| | nt | 82 |
| gag-L21S(ZF1(C>S)-ΔZF2):HiBiT:PCP | aa | 2 |
| | nt | 83 |
| gag(ZF1(C>S)-ΔZF2):HiBiT:NLS-NES-NLS:PCP | aa | 3 |
| | nt | 84 |
| gag(ZF1(C>S)-ΔZF2):HiBiT:PCP | aa | 4 |
| | nt | 85 |
| gag(ZF1(C>S)-ΔZF2):HiBiT | aa | 5 |
| | nt | 86 |
| gag-L21S(ZF1(C>S)-ΔZF2):HiBiT | aa | 6 |
| | nt | 87 |
| minimal-gag(new-codons)_(Δ12-114_Δ133-277_ΔNC-p1)_GCN4:HiBit:PCP:p6 | aa | 12 |
| | nt | 93 |
| minimal-gag(new-codons)_(Δ12-114_Δ133-277_ΔNC-p1)_GCN4:HiBit:NLS-NES-NLS:PCP:p6 | aa | 13 |
| | nt | 94 |
| minimal-gag(new-codons)_(Δ12-114_Δ133-277_ΔNC-p1)_GCN4:HiBit:KKKKNG-NLS-NES:PCP:p6 | aa | 14 |
| | nt | 95 |
| minimal-gag(new-codons)_(Δ12-114_Δ133-277_ΔNC-p1)_GCN4:HiBit:SV40-NLS-NES:PCP:p6 | aa | 15 |
| | nt | 96 |
| minimal-gag(new-codons)_(Δ12-114_ΔNC-p1)_GCN4:HiBit:PCP:p6 | aa | 16 |
| | nt | 97 |
| minimal-gag(new-codons)_(Δ12-114_ΔNC-p1)_GCN4:HiBit:NLS-NES-NLS:PCP:p6 | aa | 17 |
| | nt | 98 |
| PLC-PHP:minimal-gag(new-codons)_(Δ12-114_Δ133-277_ΔNC-p1)_GCN4:HiBit:PCP:p6 | aa | 18 |
| | nt | 99 |
| PLC-PHP:minimal-gag(new-codons)_(Δ12-114_Δ133-277_ΔNC-p1)_GCN4:HiBit:NLS-NES-NLS:PCP:p6 | aa | 19 |
| | nt | 100 |
| PLC-PHP:minimal-gag(new-codons)_(Δ12-114_Δ133-277_ΔNC-p1)_GCN4:HiBit:KKKKLD-NLS-NES:PCP:p6 | aa | 20 |
| | nt | 101 |
| PLC-PHP:minimal-gage(new-codons)_(Δ12-114_Δ133-277_ΔNC-p1)_GCN4:HiBit:c-Myc_NLS-NES:PCP:p6 | aa | 21 |
| | nt | 102 |
| PLC-PHP:minimal-gag(new-codons)_(Δ12-114_Δ133-277_ΔNC-p1)_GCN4:HiBit:KKKKNG-NLS-NES:PCP:p6 | aa | 22 |
| | nt | 103 |
| PLC-PHP:minimal-gag(new-codons)_(Δ12-114_Δ133-277_ΔNC-p1)_GCN4:HiBit:SV40-NLS-NES:PCP:p6 | aa | 23 |
| | nt | 104 |

The features disclosed in the foregoing description, in the claims and/or in the accompanying drawings may, both separately and in any combination thereof, be material for realizing the invention in diverse forms thereof.

### REFERENCES

Accola, M.A., Strack, B., and Göttlinger, H.G. (2000). Efficient Particle Production by Minimal Gag Constructs Which Retain the Carboxy-Terminal Domain of Human Immunodeficiency Virus Type 1 Capsid-p2 and a Late Assembly Domain. Journal of Virology 74, 5395-5402. 10.1128/jvi.74.12.5395-5402.2000.
Anzalone, A.V., Randolph, P.B., Davis, J.R. et al. (2019). Search-and-replace genome editing without double-strand breaks or donor DNA. Nature 576, 149-157.
Anzalone, A.V., Gao, X.D., Podracky, C.J. et al. (2022). Programmable deletion, replacement, integration and inversion of large DNA sequences with twin prime editing. Nat Biotechnol 40, 731-740.
Banskota, S., Raguram, A., Suh, S., Du, S.W., Davis, J.R., Choi, E.H., Wang, X., Nielsen, S.C., Newby, G.A., Randolph, P.B., et al. (2022). Engineered virus-like particles for efficient in vivo delivery of therapeutic proteins. Cell 185, 250-265.e16.
Boutant, E., Bonzi, J., Anton, H., Nasim, M. B., Cathagne, R., Réal, E., & Bernacchi, S. (2020). The two zinc fingers in the nucleocapsid domain of the HIV-1 Gag precursor are equivalent for the interaction with the genomic RNA in the cytoplasm, but not for the recruitment of the complexes at the plasma membrane. bioRxiv, 2020-01.
Choi, J., Chen, W., Suiter, C.C. et al. (2022) Precise genomic deletions using paired prime editing. Nat Biotechnol 40, 218-226.
Grigorov, B., Décimo, D., Smagulova, F., Péchoux, C., Mougel, M., Muriaux, D., & Darlix, J. L. (2007). Intracellular HIV-1 Gag localization is impaired by mutations in the nucleocapsid zinc fingers. Retrovirology, 4, 1-12.
Hamilton, J. R., Tsuchida, C. A., Nguyen, D. N., Shy, B. R., McGarrigle, E. R., Espinoza, C. R. S., ... & Doudna, J. A. (2021). Targeted delivery of CRISPR-Cas9 and transgenes enables complex immune cell engineering. Cell reports, 35(9).
Hendel, A., Bak, R., Clark, J. et al. Chemically modified guide RNAs enhance CRISPR-Cas genome editing in human primary cells. Nat Biotechnol 33, 985-989 (2015).
Horns, F., Martinez, J.A., Fan, C., Haque, M., Linton, J.M., Tobin, V., Santat, L., Maggiolo, A.O., Bjorkman, P.J., Lois, C., et al. (2023). Engineering RNA export for measurement and manipulation of living cells. Cell 186, 3642-3658.e32.
Komor, A.C., Kim, Y.B., Packer, M.S., Zuris, J.A., and Liu, D.R. (2016). Programmable editing of a target base in genomic DNA without double-stranded DNA cleavage. Nature 533, 420-424.
Lu, B., Javidi-Parsijani, P., Makani, V., Mehraein-Ghomi, F., Sarhan, W.M., Sun, D., Yoo, K.W., Atala, Z.P., Lyu, P., and Atala, A. (2019). Delivering SaCas9 mRNA by lentivirus-like bionanoparticles for transient expression and efficient genome editing. Nucleic Acids Res. 47, e44.
Lyu, P., Javidi-Parsijani, P., Atala, A., and Lu, B. (2019). Delivering Cas9/sgRNA ribonucleoprotein (RNP) by lentiviral capsid-based bionanoparticles for efficient "hit-and-run" genome editing. Nucleic Acids Res. 47, e99.
Ma H, Tu LC, Naseri A, Huisman M, Zhang S, Grunwald D, Pederson T. CRISPR-Cas9 nuclear dynamics and target recognition in living cells. J Cell Biol. 2016 Aug 29;214(5):529-37.
Mangeot, P.E., Risson, V., Fusil, F. et al. Genome editing in primary cells and in vivo using viral-derived Nanoblades loaded with Cas9-sgRNA ribonucleoproteins. Nat Commun 10, 45 (2019).
Nelson, J.W., Randolph, P.B., Shen, S.P. et al. Engineered pegRNAs improve prime editing efficiency. NatBiotechnol 40, 402-410 (2022).
Sherer, N.M., Swanson, C.M., Papaioannou, S., and Malim, M.H. (2009). Matrix mediates the functional link between human immunodeficiency virus type 1 RNA nuclear export elements and the assembly competency of Gag in murine cells. J. Virol. 83, 8525-8535.
Truong, D.-J.J., Armbrust, N., Geilenkeuser, J., Lederer, E.-M., Santl, T.H., Beyer, M., Ittermann, S., Steinmaßl, E., Dyka, M., Raffl, G., et al. (2022). Intron-encoded cistronic transcripts for minimally invasive monitoring of coding and non-coding RNAs. Nat. Cell Biol. 24, 1666-1676.
Truong, D.-J.J., Armbrust, N., Geilenkeuser, J., and Westmeyer, G.G. (2023). Generation of cell lines for the usage of Intron-encoded Scarless Programmable Extranuclear Cistronic Transcripts (INSPECT). Research Square. 10.21203/rs.3.pex-2000/v1.
Truong, DJ.J., Phlairaharn, T., Eßwein, B. et al. Non-invasive and high-throughput interrogation of exon-specific isoform expression. Nat Cell Biol 23, 652-663 (2021).
Wang H, Nakamura M, Abbott TR, Zhao D, Luo K, Yu C, Nguyen CM, Lo A, Daley TP, La Russa M, Liu Y, Qi LS. CRISPR-mediated live imaging of genome editing and transcription. Science. 2019 Sep 20;365(6459):1301-1305.
Yarnall MTN, Ioannidi EI, Schmitt-Ulms C, Krajeski RN, Lim J, Villiger L, Zhou W, Jiang K, Garushyants SK, Roberts N, Zhang L, Vakulskas CA, Walker JA 2nd, Kadina AP, Zepeda AE, Holden K, Ma H, Xie J, Gao G, Foquet L, Bial G, Donnelly SK, Miyata Y, Radiloff DR, Henderson JM, Ujita A, Abudayyeh OO, Gootenberg JS. (2023) Drag-and-drop genome insertion of large sequences without double-strand DNA cleavage using CRISPR-directed integrases. Nat Biotechnol. 2023 Apr;41(4):500-512. doi: 10.1038/s41587-022-01527-4.
Zalatan JG, Lee ME, Almeida R, Gilbert LA, Whitehead EH, La Russa M, Tsai JC, Weissman JS, Dueber JE, Qi LS, Lim WA. Engineering complex synthetic transcriptional programs with CRISPR RNA scaffolds. Cell. 2015 Jan 15;160(1-2):339-50. doi: 10.1016/j.cell.2014.11.052.

## Claims

1. A protein-ribonucleic acid complex comprising
(I) at least one multi-functional protein or a set of at least two proteins, wherein the at least one multi-functional protein or the set of at least two proteins comprise the following protein domains:
*(a)* at least one plasma membrane-interacting domain;
*(b)* at least one oligomerization domain;
*(c)* a membrane-bending domain;
*(d)* at least one shuttling domain comprised of at least one nuclear export signal (NES), and
nuclear localization signal (NLS);
*(e)* a nucleic acid-binding (NAB) domain, capable of binding a single strand or double-strand RNA or DNA motif; and
*(f)* a budding domain, which comprises a monopartite or multipartite motif that enables ESCRT-dependent or ESCRT-independent budding;
*(g)* optionally, a reverse transcriptase domain;
*(h)* optionally, a maturation factor;
*(i)* optionally, a reporter-signal generating domain;
*(j)* optionally one nucleic acid-stabilizing domain; and
(II) a nucleic acid editor complex, which comprises an mRNA, a guide RNA or a ncRNA,
which is bound via the nucleic acid-binding domain *(e)* of (I), preferably as ribonucleoprotein complex, wherein only the RNA species comprises a nucleic acid motif, bound by the NAB domain *(e)* of (I), wherein the RNA species is preferentially a gRNA, and wherein the RNA species comprises optionally an autonomous 3'-stabilization motif and/or a motif that is protected by an additional heterologously expressed nucleic acid-stabilizing domain *(j)* of (I).

2. The complex of claim 1, wherein the plasma membrane-interacting domain *(a)* of (I) is:
a lipid group,
such as C12 to C18 fatty acid(s) (preferably myristoyl (C14), palmitoyl (C16)) or isoprenoid(s) (preferably farnesyl); or
a polypeptide domain capable of binding membrane proteins or membrane components,
such as pleckstrin homology (PH) domain, C2 domain, FYVE domain, phox homology (PX) domains, PHD finger domain, PROPPINs domain, epsin N-terminal homology (ENTH) domain, BAR (Bin-Amphiphysin-Rvs) domains, Tubby domain,
and/or wherein the oligomerization domain *(b)* of (I) is a homo- and/or hetero-multimerizing domain, such as coiled coils, PDZ domains, *de novo* generated homo/hetero-multimerization domains composed of coil-only, sheet-only, or mixed-type proteins,
and/or wherein the membrane-bending domain *(c)* of (I) induces plasma membrane bending and formation of nanospheres, preferably with a diameter in the range from about 50 nm to about 1 µm,
and/or wherein the NES *(d)* of (I) is preferably the NES of HIV-1 (NES_{HIV-1}) or DBR1, more preferably a NES comprising an amino acid sequence according to SEQ ID NO: 65,
and/or wherein the NLS *(d)* of (I) is preferably NLS of c-Myc protein (NLS_{c-Myc}), NLS of SV40 (NLS_{SV40}) or a synthetic NLS, such as attenuated variants of NLS_{c-Myc} and NLS_{SV40}, or other mono- or bipartite synthetic NLS, more preferably wherein the NLS comprises an amino acid sequence according to any one of SEQ ID NOs: 48 to 54,
and/or wherein the nucleic acid-binding domain *(e)* of (I) is a C4-aptamer binding protein (Csy4-enzyme) domain, preferably a Csy4 domain comprising an amino acid sequence according to SEQ ID NO: 64, an RNA-binding PP7 coat protein (PCP) domain, preferably a PCP domain comprising an amino acid sequence according to SEQ ID NO: 77, 78, or 79, an MS2 coat protein (MCP) domain, preferably a MCP domain comprising an amino acid sequence according to SEQ ID NO: 58, a boxB domain, the N Protein of bacteriophage P22, a Com-com domain, or a psi-binding nucleocapsid (NC) domain of HIV-1 Gag, optionally wherein all domains can be arranged as a single chain tandem protein,
and/or wherein the budding domain *(f)* of (I) is a p6 domain, preferably a p6 domain comprising an amino acid sequence according to SEQ ID NO: 75, a p9 domain, a p12 domain, a p19 domain, an L domain, an M protein, an M2 protein, an F 13 protein, or a Z protein,
and/or wherein the complex comprises a reverse transcriptase domain *(g)* of (I), and an integrase domain, which is inactivated by mutating at least one of the amino acids at position D64 (such as pol D64V), D116, E152, and/or F185 of a pol protein of HIV-1.

3. The complex of claim 1 or 2, wherein (I) is a multi-functional protein, preferably based on group-specific antigen (gag), more preferably HIV-1 gag, and/or a gag-pol polyprotein, or a mini-Gag variant,
which preferably comprises any one of the following structures:
| | | |
|---|---|---|
| (i) | Gag: | *N*-myr-[MA]-[CA]-p2-[NC]-p1-[p6] |
| (ii) | Gag-pol _{D64v}: | *N*-myr-[MA]-[CA]-p2-[NC]-p1-[p6*]-PR-RT-INT_{D64v} |
| (iii) | Gag_{L21S}: | *N*-myr-[MA_{L21S}]-[CA]-p2-[NC]-p1-[p6] |
| (iv) | Gag_{ΔZF+L21S}-PCP: | *N*-myr-[MA_{L21S}]-[CA]-p2-[NC_{ZF1mutΔZF2}-PCP]-p1-[p6] |
| (v) | Gag_{ΔZF+L21S/NLS-NES}-PCP: | *N*-myr-[MA_{L21S}]-[CA]-p2-[NC_{ZF1mutΔZF2+NLS-NES}-PCP]-p1-[p6] |
| (vi) | miniGag_{ΔZF+L21SINLS-NES}-PCP: | *N*-myr-[MA_{Δ12-114}]-[CA_{Δ133-277}]-p2-CC-NLS-NES-PCP-[p6] |
| (vii) | PHminiGag_{ΔZF+L21S/NLS-NES}-PCP: | PH-[MA_{Δ12-114}]-[CA_{Δ133-277}]-p2-CC-NLS-NES-PCP-[p6], |
wherein the multi-functional protein more preferably comprises an amino acid sequence selected from any one of SEQ ID NOs: 1, 2, 13, 19, 24, 25, 26, 27, 60, and 61.

4. The complex of any one of claims 1 to 3, wherein (I) is a set of at least two proteins, which preferably comprises an amino acid sequence selected from any one of SEQ ID NOs: 26, 27, 56, 57 and 66.

5. The complex of any one of claims 1 to 4, wherein the nucleic acid editor complex (II) comprises a guide RNA, preferably a sgRNA or a pegRNA, which comprises the nucleic acid motif, bound by NAB domain *(e)* of (I) and an apo-protein of the nucleic acid editor complex, wherein the guide RNA is preferentially selected from a nucleic acid sequence according to any one of SEQ ID NOs: 160 to 169, and the apo-protein is preferentially selected from an amino acid sequences according to any one of the SEQ ID NOs: 28 to 35,
and/or wherein the nucleic acid editor complex (II) is a gene-editing ribonucleoprotein (RNP) complex, which comprises gene-editing polypeptide(s) or protein(s),
preferably CRISPR/Cas9 effectors, more preferably a CRISPR-associated endonuclease (Cas9 nuclease or Cas13),
TALEs,
pumilio proteins,
*de novo* DNA/RNA binding proteins,
or mutants thereof,
and wherein the gene-editing polypeptide(s) or protein(s) preferably further comprise a nuclear localization signal (NLS), preferably NLS of c-Myc protein (NLS_{c-Myc}), NLS of SV40 (NLS_{SV40}) or a synthetic NLS, such as attenuated variants of NLS_{c-Myc} and NLS_{SV40}, or other mono- or bipartite synthetic NLS, more preferably wherein the NLS comprises an amino acid sequence according to any one of SEQ ID NOs: 48 to 54,
or a nuclear export signal (NES), preferably the NES of HIV-1 (NES_{HIV-1}) or DBR1, more preferably a NES comprising an amino acid sequence according to SEQ ID NO: 65,
or combinations thereof.

6. The complex of any one of claims 1 to 5, wherein the RNA species comprises an autonomous 3'-stabilization motif, preferably truncated evopreQ1 comprising a ribonucleic acid sequence according to SEQ ID NO: 171, and/or a motif comprising a ribonucleic acid sequence according to SEQ ID NO: 216 that is protected by an additional heterologously expressed nucleic acid-stabilizing domain *(j)* of (I), preferably C4/Csy4 comprising an amino acid sequence according to SEQ ID NO: 64, the N-terminal domain of rotavirus NSP3 comprising an amino acid sequence according to SEQ ID NO: 323, wherein the N-terminal domain of rotavirus NSP3 optionally protects 3'-gacc RNA ends, or combinations thereof.

7. A polynucleotide encoding the multi-functional protein (I) as defined in any one of claims 1 to 6,
preferably comprising a nucleotide sequence selected from any one of SEQ ID NOs: 82 to 106,
or a set of polynucleotides encoding the set of at least two proteins (I) as defined in any one of claims 1 to 6, preferably comprising the nucleotide sequence of SEQ ID NO: 107 (psPAXD64V) and a nucleotide sequence selected from any one of SEQ ID NOs: 82 to 106.

8. Use of a polynucleotide or a set of polynucleotides of claim 7 for
- generating a nucleic acid delivery system, preferably a virus-like particle (VLP),
- delivering nucleic acids, preferably gene-editing ribonucleoproteins, epigenome modulator nucleic acids, transcriptome-editing nucleic acids, and/or epitranscriptome modulator nucleic acids.

9. A nucleic acid delivery system, comprising
*(A)* at least one multi-functional protein or a set of at least two proteins as defined in any one of claims 1 to 6;
*(B)* a gene-editing ribonucleoprotein (RNP) complex as defined in claim 1 or 5;
*(C)* a fusogenic (glyco)protein (envelope protein) or protein complex comprising at least one multi-functional protein or a set of at least two proteins, wherein the multifunctional domain or the set of at least two proteins comprise a receptor-targeting and a fusogenic domain to fuse the VLP to the target cell membrane;
*(D)* a targeting domain, such as a glycoprotein or engineered variants thereof;
*(E)* a moiety mediating endosomal escape, such as a glycoprotein or engineered variants thereof;
*(F)* optionally, a reporter-signal generating gene product, such as a luciferase or a fluorescent protein, and
*(G)* optionally, a nucleic acid-stabilizing protein, preferably Csy4, further optionally wherein the nucleic acid delivery system is a virus-like particle (VLP).

10. A plurality of polynucleotides, comprising
*(1)* a first polynucleotide or a set of polynucleotides of claim 7;
*(2)* a second polynucleotide comprising a nucleic acid sequence encoding a gene editor protein;
*(3)* a third polynucleotide comprising a nucleic acid sequence encoding a guide RNA, such as a sgRNA or pegRNA, which is optionally tagged by a nucleic acid binding domain; and
*(4)* a fourth polynucleotide comprising a nucleic acid sequence encoding an envelope protein,
or one or more vectors comprising the plurality of polynucleotides, wherein the one or more vectors are preferably three vectors.

11. A pharmaceutical composition comprising:
the polynucleotide or the set of polynucleotides of claim 7, the nucleic acid delivery system of claim 9, or the plurality of polynucleotides or the one or more vectors of claim 10;
optionally, pharmaceutically acceptable excipients and/or carriers.

12. A cell or a cell line comprising:
the multi-functional protein (I) as defined in any one of claims 1 to 6 or the set of at least two proteins (I) as defined in any one of claims 1 to 6, or the protein-ribonucleic acid complex of any one of claims 1 to 6;
or
the polynucleotide or the set of polynucleotides of claim 7;
or
the nucleic acid delivery system, preferably the VLP, of claim 9;
or
the plurality of polynucleotides or the one or more vectors of claim 10;
wherein the cell or the cell line is preferably a mammalian cell or cell line, more preferably a human cell or cell line, optionally wherein the cell or cell line can be co-treated with exogenous compounds, such as by means of lipofection, electroporation, photoporation, sonoporation, particle bombardment, microinjection, magnetofection, viral transduction, cell-penetrating peptides, calcium-phosphate, nanoparticles, or combinations thereof,
wherein the exogenous compounds are preferably selected from synthetic nanoparticles, fluorophores, small pharmaceutical compounds, positron emission tomography (PET) tracers, peptides, proteins, lipids, co-factors, small biomolecules, and combinations thereof.

13. A kit comprising:
the polynucleotide of or the set of polynucleotides of claim 7, and/or
the nucleic acid delivery system of claim 9, and/or
the plurality of polynucleotides of claim 10, and/or
one or more vectors of claim 10, and/or
the pharmaceutical composition of claim 11, and/or
the cell or cell line of claim 12.

14. A nucleic acid delivery system, preferably a virus-like particle, produced by transfecting, transducing, electroporating, lipofecting, photoporating, sonoporating, particle bombardment, microinjecting, magnetofecting, or otherwise inserting the plurality of polynucleotides or the one or more vectors of claim 10 into a cell and expressing the components of the nucleic acid delivery system from the plurality of polynucleotides or the one or more vectors in the cell, thereby allowing the nucleic acid delivery system, preferably the virus-like particle, to assemble in the cell.

15. The nucleic acid delivery system, preferably a virus-like particle, of claim 9 or 14, or the cell or cell line of claim 12 for use in medicine.

16. The nucleic acid delivery system, preferably a virus-like particle, of claim 9 or 14, or the cell or cell line of claim 12 for use in the treatment of genetic diseases, preferably monogenetic diseases and/or polygenetic diseases.

17. Use of a cell or cell line of claim 12 for
- *ex vivo* generating a therapeutic cell line, a diagnostic cell line, or a cell line for tissue engineering,
- tissue engineering,
- preclinical applications, and/or
- generating a VLP-distributing cell line, which preferably can be implanted into a combination of cells, a tissue and/or organism to produce VLPs therein.

18. An *in vitro* or *in vivo* method of editing a nucleic acid molecule in a target cell by nuclease mediated editing, base editing or prime editing, or homology directed repair, such as by co-delivery of an integrase-deficient (D64V) lentivirus (IDLV), wherein, preferably, the IDLV is packaged using a psi-NC interaction, a PP7-PCP, MS2-MCP, and/or a C4-Csy4 interaction, comprising:
contacting the target cell with a nucleic acid delivery system of claim 9 or 14, or with the pharmaceutical composition of claim 11,
thereby installing one or more modifications to the nucleic acid at a target site;
optionally wherein the target cell is a mammalian cell, preferably a human cell;
or wherein the target cell is in a subject, preferably a mammal, more preferably a human;
further optionally wherein the one or more modifications to the nucleic acid are associated with reducing, relieving, or preventing symptoms of a disease or disorder.

19. A method for nucleic acid delivery, preferably gene-editing nucleic acids, comprising the steps of:
(I) providing the multi-functional protein (I) as defined in any one of claims 1 to 6 or the set of at least two proteins (I) as defined in any one of claims 1 to 6 to a cell, wherein said cell produces in its nucleus a gene-editing ribonucleoprotein (RNP) complex as defined in claim 1 or 5,
(II) allowing the multi-functional protein or the set of at least two proteins to locate to the nucleus of said cell;
(III) forming a complex of the multi-functional protein or the set of at least two proteins with the gene-editing ribonucleoprotein (RNP) complex in the nucleus;
(IV) allowing the complex of (III) to be exported to the cytosol of the cell;
(V) budding of virus-like particles (VLPs) comprising the gene-editing ribonucleoprotein (RNP) complex;
(VI) binding of the VLPs to a target cell surface,
preferably via a specific receptor;
(VII) internalization of the VLPs into the target cell,
preferably via endocytosis;
(VIII) transport of the internalized complex from (VII) into the cytosol,
such as via endosomal escape;
(IX) dissociation of the RNP (with bound nucleic acid) or a nucleic acid from the nucleic acid binding domain of (I);
(X) optionally, transport of the RNP into the nucleus for genome editing or transactivation, and
(XI) optionally, binding of the guide-RNA-editing complex to an RNA target sequence.
